# EUROPÄISCHE PATENTSCHRIFT

(11) **EP 3 138 849 B1**
(45) Veröffentlichungstag und Bekanntmachung des Hinweises auf die Patenterteilung: **18.03.2020**
(21) Anmeldenummer: 16169348.6
(22) Anmeldetag: 12.05.2016
(51) Int. Cl.: C07H 15/22, A61P 31/02

(54) **PHOSPHORHALTIGE AMINOGLYCOSIDANTIBIOTIKA-SALZE**
SALTS OF PHOSPHORUS-CONTAINING AMINOGLYCOSIDE ANTIBIOTICS
SEL D'ANTIBIOTIQUES AMINOGLYCOSIDIQUES CONTENANT DU PHOSPHORE

(30) Priorität: 31.07.2015 DE 102015214603
(43) Veröffentlichungstag der Anmeldung: 08.03.2017
(73) Patentinhaber: Mathys AG Bettlach, 2544 Bettlach (CH)
(72) Erfinder: Witt, Carolin, 07747 Jena (DE); Oberbach, Thomas, 07629 Reichenbach (DE)
(74) Vertreter: Körfer, Thomas

(56) Entgegenhaltungen:
- EP-A1- 0 065 123
- WO-A2-95/34571
- DE-A1- 3 431 534
- DE-A1- 10 142 465
- US-A- 5 409 704
- M P Mingeot-Leclercq ET AL: "Aminoglycosides: activity and resistance", Antimicrobial agents and chemotherapy, Bd. 43, Nr. 4 1. April 1999 (1999-04-01), Seiten 727-737, XP055338602, UNITED STATES Gefunden im Internet: URL:https://www.google.de/url?sa=t&rct=j&q =&esrc=s&source=web&cd=&sqi=2&ved=0ahUKEwj jzefv9dzRAhXGnBoKHYG9BaUQFgg3MAI&url=http: //citeseerx.ist.psu.edu/viewdoc/download?d oi=10.1.1.563.1344&rep=rep1&type=pdf&usg=A FQjCNFGPtziDtv0uwjnWrVF1W7CXACgoQ&bvm=bv.1 45063293,d.d2s [gefunden am 2014-02-27]

## Beschreibung

### GEBIET DER ERFINDUNG

Beschrieben wird die Synthese von Aminoglycosid-Salzen durch Umsetzung von Aminoglycosid-Antibiotika oder Aminoglycosidgruppen-haltigen Antibiotika mit Alkylgruppen-haltigen (HO)ₙP(O)-Säuren. Die Synthese ermöglicht die Herstellung von in Wasser wenig löslichen Aminoglycosid-Salzen. Die Erfindung dient der Bereitstellung Aminoglycosid-haltiger Salze zur Anwendung in der Human- und Veterinärmedizin, Medizintechnik, Biologie sowie in der pharmazeutischen Industrie.

### HINTERGRUND DER ERFINDUNG

Die Behandlung bakterieller Infektionen im Weich- und Hartgewebebereich in der Human- und Veterinärmedizin erfordert hohe lokale Antibiotika-Konzentrationen. Es ist bekannt, dass die systemische Applikation von Antibiotika zum Erreichen lokaler therapeutisch relevanter Wirkstoffspiegel mit hohen Antibiotika-Dosen und der damit einhergehenden hohen Wirkstoffbelastung des Gesamtorganismus mit einer Reihe von Problemen, insbesondere mit starken Nebenwirkungen, verbunden ist. Daher ist es vorteilhaft, Antibiotika in lokal applizierbaren Freisetzungssystemen anzuwenden oder Antibiotika in geeignete Depotformen zu überführen.

Depotsysteme zur verzögerten Freisetzung von Antibiotika zur Behandlung von lokalen Infektionen sind bekannt. Dabei können die Antibiotika an die Oberfläche eines Trägers absorptiv gebunden sein oder in einer nichtresorbierbaren bzw. resorbierbaren Matrix vorliegen.

Die Synthese von leicht wasserlöslichen Aminoglycosid-Salzen anorganischer Säuren wie Salz-, Schwefel- und Phosphorsäure sowie organischen Säuren, die in Abhängigkeit des organischen Restes unterschiedliche Löslichkeit in Wasser und organischen Lösungsmitteln aufweisen, ist bereits bekannt.

Die DE 34 31 534 A1 offenbart Verbindungen mit einer Flavonoid-Grundstruktur, die neben den Hydroxyl-Gruppen am Phenyl-Ring, der an den Oxan-Ring gebunden ist, zusätzlich eine aromatische Ketogruppe und eine Ethylgruppe trägt, an welcher der aromatische oder aliphatische Rest 2 (Ar2) gebunden ist.

Die EP 0 065 123 A1 beschreibt schwerlösliche Salze aus Flavonon- bzw. Flavonderivaten mit Aminoglycosiden. Für geeignete Dosierungsformen werden Träger- oder Hilfsstoffe benötigt.

In der WO 95/34571 A2 geht es um ein Freisetzungssystem aus biologisch aktiven organischen Wirkstoffen, Polysulfon- bzw. Polyphophonsäuren von Sacchariden und einem physiologisch tolerierbaren Gegenion. Die Stoffe können zusammen mit anderen Hilfsstoffen bevorzugt peroral in Form von Pulver, Kapseln, Tabletten oder als Aerosole, Pflaster, Pasten, Cremes oder Emulsionen verabreicht werden.

Die US 5 409 704 A offenbart Phosphatsalze von Aminoglycosid-Antibiotika, die in ein Liposom eingebracht sind.

M P Mingeot-Leclercq et al.: "Aminoglycosides: Activity and Resistance", Antimicrobial Agents and Chemotherapy, vol. 43(4), 1. April 1999, Seiten 727-737, bezieht sich auf verschiedene Aminoglycoside und deren antimikrobielle Aktivität.

In keinem der zitierten Dokumente wird jedoch eine retardierende Wirkstofffreisetzung über mehrere Wochen beschrieben. Des Weiteren wird nicht auf die Haftungseigenschaften eingegangen.

In DE 101 42 465 A1 wird ein Verfahren zur antibiotischen Beschichtung nichtmetallischer Körper mit interkonnektierenden Mikrohohlräumen, ein entsprechend beschichteter Körper mit interkonnektierenden Mikrohohlräumen und dessen Verwendung als Implantat offenbart. Insbesondere wird die antibiotische Beschichtung von porösen Oberflächen mit Antibiotika-Sulfaten oder -Sulfonaten dargestellt.

In US 4,617,293 werden Flavanoidphosphate von Aminoglycosid-Antibiotika als Wirkstoff-Depot beschrieben.

In den bekannten Antibiotika-Depotsystemen ist die verzögerte / retardierende Wirkstofffreisetzung physikalisch begründet und stark von der Zusammensetzung und der Struktur der verwendeten Matrix abhängig. Weiterhin ist der Herstellungsprozess dieser Antibiotika-Systeme von nicht unerheblichem Einfluss auf das Freisetzungsverhalten.

Die bisher bekannten Antibiotika-Depotsysteme weisen einen starken Burst release von nicht porösen Oberflächen, also eine schlagartigen Wirkstofffreisetzung in das umgebende Medium oder das umliegende Gewebe auf, wodurch ein Großteil des eingesetzten Wirkstoffs schnell von der Infektionsstelle abtransportiert wird. Somit ist das Antibiotika-Depot zum Erreichen therapeutisch relevanter Wirkstoffspiegel nur für kurze Zeit verfügbar.

Durch die Verwendung von Sulfaten oder -Sulfonaten als Depotsystem kann es ferner zu Gesundheitsrisiken für den Organismus kommen, insbesondere können diese Verbindungen allergische und / oder reizende Reaktionen hervorrufen.

Von diesem Stand der Technik ausgehend ist es Aufgabe der vorliegenden Erfindung auf geeignete Art und Weise in Wasser wenig lösliche Aminoglycosid-Verbindungen und Zusammensetzungen mit retardierender Wirkstofffreisetzung zu entwickeln, die einen geringen oder kontrollierbaren Burst release und langfristig (bis zu mehreren Wochen) konstante Wirkstoffspiegel in therapeutisch wirksamen Konzentrationen aufweisen. Ferner soll deren Verwendung sowie ein geeignetes Herstellungsverfahren bereitgestellt werden.

Die erfindungsgemäßen Verbindungen und Zusammensetzungen sollen in der Human- und Veterinärmedizin zur Behandlung lokaler mikrobieller Infektionen im Knochen- und Weichgewebebereich als Wirkstoff-Depot oder als antibakterielle Beschichtung für resorbierbare und nichtresorbierbare Implantate anwendbar sein sowie mit resorbierbaren als auch nichtresorbierbaren Hilfsstoffen unterschiedlichster Struktur zu Implantaten verarbeitet werden können, wobei die Wirkstofffreisetzung im Wesentlichen unabhängig von den Trägermaterialien sein soll.

### ZUSAMMENFASSUNG DER ERFINDUNG

Diese Aufgabe wird bezüglich der Verbindungen, Zusammensetzungen, Implantate und antibakteriellen Beschichtungen durch die Merkmale der Ansprüche 1, 11, 18 und 20 sowie im Hinblick auf das Herstellungsverfahren durch die Merkmale des Anspruchs 12 gelöst. Im Bezug auf die Verwendungen wird diese Aufgabe durch die Merkmale des Anspruchs 15 gelöst, während die Merkmale des Anspruchs 16 die Lösung der Aufgabe im Hinblick auf weitere medizinische Indikationen aufzeigen.

Die abhängigen Ansprüche enthalten vorteilhafte weitere Ausführungsformen der Erfindung.

Überraschend wurde gefunden, dass die Umsetzung von Aminoglycosiden mit Alkyl-haltigen ein- und mehrbasigen (HO)ₙP(O)-Säuren zu in Wasser wenig löslichen Salzen führt, die sich in organischen Lösungsmitteln oder Lösungsmittelgemischen lösen und deren Aminoglycosid-Gehalt über das molare Verhältnis zur eingesetzten Säure eingestellt werden kann.

Sehr überraschend war der Befund, dass diese Salze einen geringen Burst release zeigen und Aminoglycosid-Antibiotika in konstanten, therapeutisch wirksamen Konzentrationen über einen Zeitraum von bis zu mehreren Wochen freisetzen.

Überraschenderweise zeichnen sich derartige Verbindungen durch retardierende Freisetzung eines Aminoglykosid-Antibiotikums sowohl von glatten als auch rauen, strukturierten Oberflächen aus.

Ebenfalls sehr überraschend wurde gefunden, dass die Verbindungen gemäß der vorliegenden Erfindung nicht nur eine starke antibakterielle Wirkung sondern auch einen wachstumsfördernden Effekt auf Körperzellen, insbesondere Knochenzellen, aufweisen.

Aufgrund dieses ausgezeichneten Freisetzungsprofils und der zellwachstumsfördernden Eigenschaften sind die erfindungsgemäßen Aminoglycosid-Verbindungen sehr gut für Beschichtungen mit retardierender Aminoglycosid-Freisetzung geeignet, beispielsweise zum Beschichten von Implantaten.

Aminoglycosid-Verbindungen mit Alkylgruppen-haltigen ein- und mehrbasigen (HO)ₙP(O)-Säuren sind bisher nicht bekannt.

Vorteilhaft wird die Aufgabe durch eine Verbindung der Formel 1 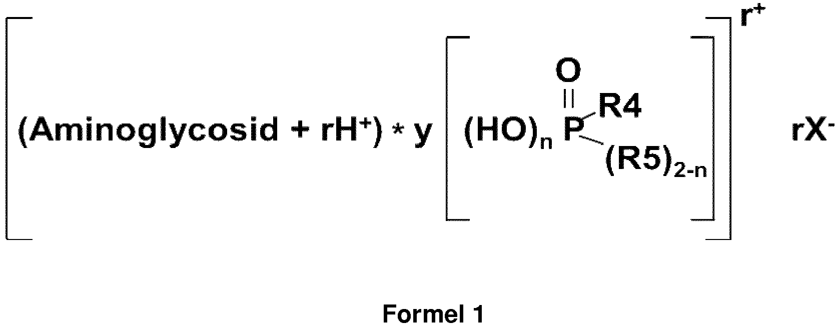 gelöst, wobei das Aminoglycosid ausgewählt ist aus der Gruppe der Aminoglycosid- oder Aminoglycosidgruppen-haltigen Antibiotika, wobei y ausgewählt ist aus 0,1 bis 20, wobei n ausgewählt ist aus 1 oder 2, wobei r ausgewählt ist aus 0, 1, 2 bis 5, wobei X ausgewählt ist aus der Gruppe bestehend aus Halogenen, HCOO, CH₃COO, CH₃SO₃, Phosphatresten, oder Sulfatresten, wobei R4 und R5 unabhängig ausgewählt sind aus der Gruppe bestehend aus -C₁-C₃₀-Alkyl, -C₂-C₃₀-Alkenyl, -C₂-C₃₀-Alkinyl, -O-(C₄-C₃₀-Alkandiyl)phosphat, -(C₄-C₃₀-Alkandiyl)phosphonat, -O-R6, -O-P(O)(OH)(O-R6), -O-P(O)(-O-R6)(-O-R7), -O-P(O)(-R6)(-O-R7), -O⁻[NR8,R9,R10,H]⁺, -O-P(O)(-R6)(-O⁻[NR8,R9,R10,H]⁺), -O-P(O)(-O-R6)(-O⁻[NR8,R9,R10,H]⁺), wobei R6, R7, R8, R9 und R10 unabhängig ausgewählt sind aus der Gruppe - C₁-C₃₀-Alkyl, -C₂-C₃₀-Alkenyl oder -C₂-C₃₀-Alkinyl.

Bevorzugt sind R4 und R5 unabhängig voneinander ausgewählt aus der Gruppe bestehend aus -C₆-C₂₆-Alkyl, -C₆-C₂₆-Alkenyl, -C₆-C₂₆-Alkinyl.

Besonders bevorzugt sind R4 und R5 unabhängig voneinander ausgewählt aus der Gruppe bestehend aus -C₆-C₂₀-Alkyl, -C₆-C₂₀-Alkenyl, -C₆-C₂₀-Alkinyl.

Weiter vorteilhaft sind R6, R7, R8, R9 und R10 unabhängig voneinander ausgewählt aus der Gruppe bestehend aus -C₆-C₂₆-Alkyl, -C₆-C₂₆-Alkenyl, -C₆-C₂₆-Alkinyl.

Vorzugsweise bedeutet y in Formel 1 0.33, 1, 2, 2.5, 3, 4 oder 5.

Weiter bevorzugt ist X in Formel 1 ausgewählt aus Chlor, Fluor, Iod oder Brom, insbesondere aus Chlor oder Brom.

Vorteilhaft enthalten die Gruppen -C₁-C₃₀-Alkyl, -C₂-C₃₀-Alkenyl, -C₂-C₃₀-Alkinyl in Formel 1 zwei oder mehrere ungesättigte C-Atome, sind verzweigt oder unverzweigt, oder mit Halogenatomen oder organischen funktionellen Gruppen substituiert. Besonders bevorzugt sind Halogenatome wie Fluor oder Chlor sowie Hydroxyl-, Ester-, Ether- oder Carbonsäuregruppen.

Vorzugsweise enthalten die Gruppen -C₆-C₂₆-Alkyl, -C₆-C₂₆-Alkenyl, -C₆-C₂₆-Alkinyl zwei oder mehrere ungesättigte C-Atome, sind verzweigt oder unverzweigt, oder mit Halogenatomen, vorzugsweise Fluor oder Chlor, oder organischen funktionellen Gruppen substituiert.

Weiter bevorzugt enthalen die Gruppen -C₆-C₂₀-Alkyl, -C₆-C₂₀-Alkenyl, -C₆-C₂₀-Alkinyl zwei oder mehrere ungesättigte C-Atome, sind verzweigt oder unverzweigt, oder mit Halogenatomen, vorzugsweise Fluor oder Chlor, oder organischen funktionellen Gruppen substituiert.

Weiter vorteilhaft sind R8 und R9 in Formel 1 unabhängig voneinander H.

Besonders bevorzugt ist n in Formel 1 1 und R5 H.

Das Aminoglycosid oder Aminoglycosidgruppen-haltige Antibiotikum in Formel 1 ist vorteilhaft ausgewählt aus der Gruppe bestehend aus Amikacin, Apramycin, Geneticin, Kanamycin, Netilmicin, Neomycin, Paromomycin, Spectinomycin, Streptomycin, Tobramycin, Gentamicin, oder Vancomycin.

Besonders bevorzugt ist die Verbindung gemäß Formel 1 ausgewählt aus der Gruppe bestehend aus Gentamicin-Dodecylphosphat, Genatmicin-Octadecylphosphonat, Vancomycin-Dodecylphosphat, Vancomycin-Octadecylphosphonat, Streptomycin-Dodecylphosphat, Kanamycin-Dodecylphosphat oder Kanamycin-Octadecylphosphonat. Besonders bevorzugt ist die Verbindung gemäß Formel 1 Gentamicin-Dodecylphosphat oder Gentamicin-Octadecylphosphonat.

Vorzugsweise ist das erfindungsgemäße Aminoglycosid Gentamicin entsprechend Formel 2, wobei R1, R2 und R3 unabhängig voneinander H oder eine Methylgruppe darstellen. R4 und R5 sind unabhängig voneinander ausgewählt aus der Gruppe bestehend aus -C₈-C₂₂-Alkyl, -C₈-C₂₂-Alkenyl, welche zwei oder mehrere ungesättigte C-Atome enthalten können, und verzweigt oder unverzweigt sind. Des Weiteren können R4 und R5 unabhängig voneinander eine Gruppe -O-R6, -O-P(O)(OH)(O-R6), -O-P(O)(-O-R6)(-O-R7), -O-P(O)(-R6)(-O-R7), -O⁻[NR8,R9,R10,H]⁺, -O-P(O)(-R6)(-O⁻ [NR8,R9,R10,H]⁺), -O-P(O)(-O-R6)(-O⁻[NR8,R9,R10,H]⁺) sein, wobei die R6, R7, und R10 unabhängig voneinander ausgewählt sind aus der Gruppe bestehend aus -C₁-C₂₂-Alkyl, -C₁-C₂₂-Alkenyl, welche zwei oder mehrere ungesättigte C-Atome enthalten können, verzweigt oder unverzweigt sein können und R8 und R9 H sind.

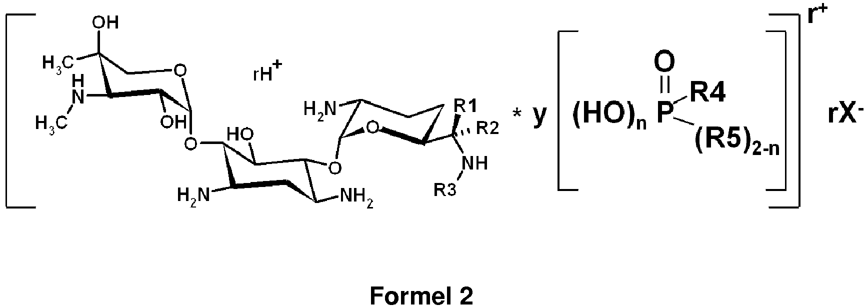

Das Aminoglycosid der erfindungsgemäßen Verbindung wird vorteilhaft retardierend freigesetzt.

Weiter von Vorteil fördert die erfindungsgemäße Verbindung das Zellwachstum. Besonders vorteilhaft fördert die erfindungsgemäße Verbindung das Wachstum von Knochenzellen.

Die erfindungsgemäße Verbindung kann vorzugsweise in gelöster oder fester Form vorliegen.

In einer zweiten Ausführungsform wird die der vorliegenden Erfindung zugrundeliegende Aufgabe durch die Bereitstellung einer Zusammensetzung, welche mindestens 2, 3, 4, 5, 6, 7, 8, 9, 10, 11, oder 12 verschiedene Verbindungen gemäß der ersten Ausführungsform umfasst, gelöst.

Vorteilhaft umfasst die erfindungsgemäße Zusammensetzung mindestens 2, 3, 4, 5, 6, 7, 8, 9, 10, 11, oder 12 verschiedene Aminoglycoside ausgewählt aus der Gruppe bestehend aus Amikacin, Apramycin, Geneticin, Kanamycin, Netilmicin, Neomycin, Paromomycin, Spectinomycin, Streptomycin, Tobramycin, Gentamicin, oder Vancomycin.

Die Verbindung gemäß der ersten Ausführungsform wird bevorzugt durch ein Verfahren, das die folgenden Schritte umfasst, hergestellt:
a) Vorlegen eines Aminoglycosids,
b) Umsetzen des Aminoglycosids mit einer Alkylgruppen-haltigen (HO)ₙP(O)-Säure, vorzugsweise Dibutylphosphat, Amylphosphat, Diamylphosphat, Octadecylphosphat, Tertadecylphosphat, Bis(tertadecyl)phosphat, Dodecylphosphat, Bis(dodecyl)phosphat, 2-Ethylhexylphosphat, Bis(2-ethylhexyl)phosphat, Decylphosphat, Bis(decyl)phosphat, Bis(undecyl)phosphat, Bis(2,6-dimethylheptan-4-yl)phosphat, Tridecanolphosphat, Hexadecenylphosphat, Hexadecylphosphat, Bis(hexadecyl)phosphat, Octadecenphosphat, Bis(octadecyl)phosphat, 9-Octadecen-1-phosphat, Docosaphosphat, Isopentenyldiphosphat, Dibutyldiphosphat, Dioctylpyrophosphat, Tris(butyl)pyrophosphat, Tris(3-methybutyl)phosphat, Tris(isooctyl)diphosphat, Tris(tridecyl)diphosphat, Mono-[2-(perfluorooctyl)ethyl]phosphat, Hexan-1,6-diyl-bis(dihydrogenphosphat), Octylphosphonsäure, Decylphosphonsäure, Butylhydrogen-2-butylphosphonat, 2-Ethylhexylhydrogen-2-ethylhexylphosphonat, Dodecylphosphonsäure, Tetradecylphosphonsäure, Octadecylphosphonsäure, Phosphonopropionsäure, P,P'-dioctadecyldiphosphonsäure, 1,4-Butandiphosphonsäure
c) Erhalten eines Aminoglycosid-Salzes.

Das Umsetzen des Aminoglycosids entsprechend Schritt b) des Verfahrens zur Herstellung einer erfindungsgemäßen Verbindung kann vorteilhaft in Gegenwart einer Base und Alkali- oder Erdalkalisalzen einer Alkylgruppen-haltigen (HO)ₙP(O)-Säure erfolgen.

Weiter bevorzugt wird in Schritt a) des erfindungsgemäßen Verfahrens ein Aminoglycosid oder ein Aminoglycosid-Salz vorgelegt und das Umsetzen mit Alkylgruppen-haltigen (HO)ₙP(O)-Säuren in Schritt b) erfolgt in Gegenwart eines Alkylamins.

Besonders bevorzugt ist die erfindungsgemäße Aminoglykosid-Verbindung durch ein Herstellungsverfahren umfassend folgende Schritte:
a) Vorlegen eines Aminoglycosids,
b) Umsetzen des Aminoglycosids mit einer Alkylgruppen-haltigen (HO)ₙP(O)-Säure,
c) Erhalten eines Aminoglycosid-Salzes,
erhältlich.

Vorteilhaft ist die erfindungsgemäße Aminoglycosid-Verbindung durch ein Verfahren erhältlich umfassend die Schritte
a) Vorlegen eines Aminoglycosids,
b) Umsetzen des Aminoglycosids in Gegenwart einer Base und Alkali- oder Erdalkalisalzen einer Alkylgruppen-haltigen (HO)ₙP(O)-Säure,
c) Erhalten eines Aminoglycosid-Salzes.

Weiter bevorzugt ist die erfindungsgemäße Aminoglycosid-Verbindung erhältlich durch ein Verfahren umfassend die Schritte:
a) Vorlegen eines Aminoglycosids oder eines Aminoglycosid-Salzes
b) Umsetzen des Aminoglycosids oder des Aminoglycosid-Salzes mit einer Alkylgruppen-haltigen (HO)ₙP(O)-Säure in Gegenwart eines Alkylamins,
c) Erhalten eines Aminoglycosid-Salzes.

Gemäß einer dritten Ausführungsform der vorliegenden Erfindung wird die Aufgabe durch Verwendung einer erfindungsgemäßen Verbindung oder Zusammensetzung a) als injizierbares, lokales Antibiotika-Depot, b) zur antibakteriellen Beschichtung von Implantaten und / oder c) zur zellwachstumsfördernden Beschichtung von Implantaten, gelöst.

Weiter bevorzugt erfolgt die Verwendung der erfindungsgemäßen Verbindung oder Zusammensetzung zur retardierenden Wirkstofffreisetzung.

In einer vierten Ausführungsform wird die erfindungsgemäße Verbindung oder Zusammensetzung als Medikament verwendet.

Vorteilhaft wird die Verbindung oder Zusammensetzung gemäß der vorliegenden Erfindung a) zur Behandlung von Infektionen und / oder b) zur Förderung des Einwachsverhaltens von Implantaten, verwendet.

Vorzugsweise wird die der Erfindung zugrundeliegende Aufgabe durch Bereitstellung eines Implantats, welches die erfindungsgemäße Verbindung oder Zusammensetzung umfasst, gelöst.

Vorteilhaft dient das erfindungsgemäße Implantat der Behandlung von Knochen- oder Weichteildefekten.

In einer weiteren Ausführungsform wird eine antibakterielle Beschichtung, die eine erfindungsgemäße Verbindung oder Zusammensetzung umfasst, bereitgestellt.

### KURZE BESCHREIBUNG DER FIGUREN DER ZEICHNUNG

In den Figuren der Zeichnung werden Ausführungsbeispiele der Erfindung beispielhaft erläutert.
- **Fig. 1**: zeigt das Freisetzungsprofil von Gentamicin-Base in [µg] (y-Achse) aus Gentamicin-Palmitat (■), Gentamicin-Dodecylphosphat (◆) und Gentamicin-Octadecylphosphonsäure (▲) in Tagen (x-Achse).
- **Fig. 2**: gibt eine mikroskopische Aufnahme der Zytokompatibilitäts-Untersuchungen von Beschichtungen mit Gentamicin-Palmitat (A), Gentamicin-Dodecylphosphat (B) und Gentamicin-Octadecylphosphat (C) nach einer Inkubationszeit von 4 Tagen wieder.

### DETAILLIERTE BESCHREIBUNG

Die vorliegende Erfindung stellt gering wasserlösliche Aminoglycosid-Verbindungen mit retardierender Wirkstofffreisetzung, einem geringen oder kontrollierbarem Burst release und langfristig (über mehrere Wochen) stabilen therapeutisch relevanten Konzentrationen bereit.

"Aminoglycoside" im Sinne der vorliegenden Erfindung sind eine heterogene Gruppe von Oligosaccharid-Antibiotika in denen Aminomonosaccharid- und Monosaccharidbausteine miteinander und / oder mit basischen, mehrere Hydroxygruppen enthaltenden Cyclohexanderivaten verknüpft sind. Sie umfassen Aminoglycosid- oder Aminoglycosidgruppen-haltige Antibiotika und werden biosynthetisch oder semisynthetisch von Streptomyces- oder Microspora-Arten gewonnen.

"Antibiotika" gemäß der vorliegenden Erfindung sind Substanzen mit antimikrobieller Wirkung, die natürlich von Pilzen oder Bakterien gebildet werden oder teilsynthetisch, vollsynthetisch oder gentechnisch gewonnen werden. Antibiotika hemmen die Stoffwechselprozesse von Mikroorganismen und verhindern dadurch deren Vermehrung und / oder Überleben.

Aminoglycosid-Antibiotika im Sinne der vorliegenden Erfindung umfassen Amikacin, Apramycin, Geneticin, Hygromycin, Kanamycin, Netilmicin, Neomycin, Paromomycin, Spectinomycin, Streptomycin, Tobramycin und vorzugsweise Gentamicin.

Erfindungsgemäß bedeuten Aminoglycosidgruppen-haltige Antibiotika Vancomycin, Teicoplanine und Telavancin, vorzugsweise Vancomycin.

Alkylgruppen-haltige (HO)ₙP(O)-Säuren bedeuten Organylphosphate, Phosphonate, Organylphosphonate, organylsubstituierte Di- und Polyphosphate, Di- und Polyphosphonate, organylsubstituierte Di- und Polyphosphonate.

C₁-C₃₀-Alkyl gemäß der vorliegenden Erfindung sind miteinander verbundene Kohlenstoff- und Wasserstoffatome. Die einfachste Alkylgruppe ist die Methylgruppe (CH₃). Andere Beispiele für Alkyle sind Ethyl, Propyl, Butyl etc.

Alkenyl im Sinne der vorliegenden Erfindung ist eine funktionelle Kohlenwasserstoffgruppe basierend auf einem Alken.

Alkinyl gemäß der Erfindung ist eine funktionelle Kohlenwasserstoffgruppe basierend auf einem Alkin.

Funktionelle Gruppen im Sinne der vorliegenden Erfindung sind Atomgruppen in organischen Verbindungen, die anstelle eines Wasserstoffs mit einer Kohlenwasserstoffkette kovalent verbunden sind und die die Stoffeigenschaften und das Reaktionsverhalten der sie tragenden Verbindung maßgeblich bestimmen. Beispiele für funktionelle Gruppen gemäß der vorliegenden Erfindung sind Halogen, OH-, Alkyl-O- (Ether-), Alkoxycarbonyl-(Ester-), Carboxylgruppen.

Die Alkylketten in der Aminoglycosid-Verbindung bestimmen maßgeblich deren Polarität (hydrophob / hydrophil) und Organolöslichkeit. Dadurch lässt sich die gewünschte Geschwindigkeit und Menge der Wirkstofffreisetzung beliebig einstellen und / oder eine geeignete Dosierung des Wirkstoffs erzielen. Somit ist auch die Stärke des Burst release kontrollierbar bzw. bestimmbar. Insbesondere kann der Burst release vermieden werden, wenn die Alkylkette eines einzelnen gebundnen anionischen Gegenions mindestens einen C₈-Rest, vorzugsweise einen C₉-, C₁₀-, C₁₁-, C₁₂-, C₁₃-, C₁₄-, C₁₅-, C₁₆-, C₁₇-, C₁₈-, C₁₉-, C₂₀-, C₂₁-, C₂₂-, C₂₃-, C₂₄-, C₂₅-, C₂₆-, C₂₇-, C₂₈-, C₂₉-, oder einen C₃₀-Rest, darstellt. Sind 2 oder mehrere Anionen gebunden sollte der organische Rest größer/gleich einem C₄-Rest, vorzusweise ein C₄-Rest, weiter bevorzugt ein C₅-, C₆-, C₇-, C₈-, C₉-, C₁₀-, C₁₁-, C₁₂-, C₁₃-, C₁₄-, C₁₅-, C₁₆-, C₁₇-, C₁₈-, C₁₉-, C₂₀-, C₂₁-, C₂₂-, C₂₃-, C₂₄-, C₂₅-, C₂₆-, C₂₇-, C₂₈-, C₂₉-, oder ein C₃₀-Rest, sein.

Je hydrophober die Aminoglycosid-Verbindung, desto langsamer wird der Wirkstoff, also das Aminoglycosid, freigesetzt.

Ferner resultiert die Organolöslichkeit der erfindungsgemäßen Verbindungen in vorteilhafter Wachsbildung. Die wachsartige Beschaffenheit erlaubt eine geeignete Beschichtung jeglicher Oberflächen (verbessertes Anhaftungsvermögen).

Beschichtungsfähig sind sowohl glatte als auch raue oder strukturierte Oberflächen. Diese können beispielsweise aus Metallen, insbesondere Edelmetallen wie Gold und Platin, oder anderen korrosionsbeständigen Metallen wie Titan oder Titanlegierungen, Kobaltbasislegierungen, ferner aus Keramik oder Kunststoff wie Polyester bestehen. Außerdem können natürliche Oberflächen bestehend aus Korallen, Collagen, Chitosan oder Alginat mit den erfindungsgemäßen Verbindungen beschichtet werden. Weitere beschichtungsfähige Oberflächen bestehen aus resorbierbaren Werkstoffen wie Polymeren aus Glykolsäuren oder Dioxanon, Hydroxylapatit oder Magnesiumlegierungen.

Die Salzbildung mit Phosphaten oder Phosphonaten führt einerseits zu vorteilhafter Wachs- / Filmbildung der erfindungsgemäßen Aminoglycosid-Verbindung, wodurch sich die Freisetzung des Wirkstoffs im Hinblick auf Geschwindigkeit und Konzentration steuern lässt.

Andererseits erhöht Phosphat oder Phosphonat in den erfindungsgemäßen Aminoglycosid-Verbindungen die Verträglichkeit für den Organismus.

Insbesondere die antibakterielle / antimikrobielle Beschichtung von Implantaten, im Speziellen im Bereich der Knochenrekonstruktion und Osteosynthese, mit erfindungsgemäßen Verbindungen führt zu gesteigerter Verträglichkeit für den Organismus. Da die Mineralisierung des Knochens von der Verfügbarkeit von Calcium und Phosphat abhängt, führt das in den Aminoglycosid-Verbindungen enthaltene Phosphat zu verbesserter Knochenmineralisierung und fördert dadurch die Knochenfestigkeit.

"Implantat" im Sinne der vorliegenden Erfindung ist ein im Körper eingepflanztes oder außen am Körper angebrachtes künstliches Material, welches dauerhaft oder zumindest langfristig dort verbleiben soll. Vom Begriff umfasst sind Endo- und Exoprothesen, sowie medizinische, plastische und funktionelle Implantate.

Medizinische Implantate unterstützen oder ersetzen Körperfunktionen. Nicht abschließende Beispiele hierfür sind implantierbare Kardioverter-Defibrillatoren, Herzschrittmacher, Hirnschrittmacher, Stents, Gefäßprothesen, Cochleaimplantate, Retina-Implantate, Zahnimplantate, Gelenkersatzimplantate, Implantate zur operativen Behandlung von Knochenbrüchen (Osteosynthesematerial), Knochenersatz für Schädeldefekte oder Arzneimitteldepots, die eine Langzeitabgabe von Wirkstoffen ermöglichen.

Plastische Implantate kommen als Ersatz für zerstörte Körperteile oder als Vergrößerung vorhandener Körperteile zum Einsatz. Hierzu zählen beispielsweise Brustimplantate.

Funktionelle Implantate finden in der Überwachung von Tieren oder Menschen Anwendung, indem RFID (radio-frequency identification)-Chips (Sender-Empfänger-Systeme zur automatischen und berührungslosen Identifikation und Lokalisierung von Objekten und Lebewesen mit Radiowellen) unter die Haut verpflanzt werden.

Retardierende Freisetzung meint erfindungsgemäß eine verzögerte / verlangsamte Freisetzung eines Wirkstoffs, vorliegend insbesondere die verzögerte Freisetzung eines Aminoglycosids. Retardierung wird verwendet um gefährliche kurzzeitig hohe Konzentrationen von Arzneistoffen im Blut zu verhindern und / oder eine möglichst lange Wirkung einer Substanz zu erreichen.

Durch die retardierende Wirkstofffreisetzung gemäß der vorliegenden Erfindung wird die Zytotoxizität vermieden und gefährliche Nebenwirkungen reduziert. Ferner ist die Verfügbarkeit therapeutisch wirksamer Konzentrationen des Aminoglycosids über einen langen Zeitraum, d.h. über einen Zeitraum von mehreren Tagen bis Wochen, gesichert.

Die biologische Aktivität der in den erfindungsgemäßen Verbindungen enthaltenen Aminoglycosid-Antibiotika wie beispielsweise Gentamicin kann mittels dem Fachmann bekannten Hemmhoftest mit Bacillus subtilis (ATCC 6633) bestimmt werden.

Die verzögerte Freisetzung des Wirkstoffs und die damit einhergehende Verfügbarkeit therapeutisch relevanter Wirkstoffspiegel über mehrere Tage bis Wochen verhindert langfristig die Ansiedlung, Vermehrung / das Überleben von Mikroorganismen.

Diese retardierende Wirkstofffreisetzung ist insbesondere im Bereich der Implantologie und rekonstruktiven Medizin (Unfallchirurgie, plastische Chirurgie) von Vorteil. Auf diesem Gebiet werden lokal besonders hohe Antibiotika-Konzentrationen benötigt, um das Anwachsen von Bakterien sowie Mikrofilmbidlung auf der Implantat-Oberfläche zu vermeiden. Durch das retardierende Freisetzungsprofil der erfindungsgemäßen Verbindungen sind therapeutisch wirksame Antibiotika-Konzentrationen lokal über einen langen Zeitraum verfügbar und reduzieren so die Infektionsgefahr.

Förderung des Zellwachstums im Sinne der vorliegenden Erfindung beinhaltet Verringerung von Zelltod (Apoptose oder Nekrose), Steigerung des Zellwachstums, Steigerung der Zellproliferation, Beschleunigung des Wachstums beschädigter Zellen, Steigerung der Zellviabilität (Zelllebensfähigkeit, Lebendzellzahl).

Erfindungsgemäß beschreibt Zytotoxizität die Fähigkeit oder Eigenschaft verschiedener Substanzen (chemische Stoffe, Viren, spezifische Immunzellen) lebende Zellen oder Gewebe zu schädigen oder zu zerstören. Die Zytotoxizität kann durch Bestimmung der Zellviabilität nach Zugabe der Substanz im Vergleich zu einer Negativkontrolle ohne die entsprechende Substanz bestimmt werden. Beispiele für übliche Testmethoden sind der Neutralrot-Test, der MTT (3-(4,5-Dimethylthiazol-2-yl)-2,5-diphenyltetrazoliumbromid)-Test, ein Lebend/tot-Assay mit Fluoresceindiazetat und GelRed® oder der Trypanblau-Test.

Die erfindungsgemäßen Aminoglycosid-Verbindungen weisen eine geringe Zytotoxizität, insbesondere im Vergleich zu Aminoglycosid-Sulfaten oder - Sulfonaten sowie Salzen von Alkylcarbonsäuren auf. Die Aminoglycosid-Verbindungen gemäß der Erfindung verhindern einen anfänglichen starken Burst release, also eine schlagartige Wirkstofffreisetzung in hohen Konzentrationen, wodurch es nicht zu außergewöhnlich hohen Wirkstoff-, insbesondere Aminoglycosid-Konzentrationen im Organismus oder dem betreffenden Gewebe kommt. Die Zellverträglichkeit wird gesteigert.

### Herstellungsverfahren

Die vorliegende Erfindung stellt ferner ein Verfahren zur Herstellung einer Verbindung gemäß Formel 1 bereit. Dieses Verfahren umfasst im Wesentlichen das Vorlegen eines Aminoglycosids und das Umsetzen dieses Aminoglycosids mit einer Alkylgruppen-haltigen (HO)ₙP(O)-Säure, woraus schließlich ein Aminoglycosid-Salz erhalten wird.

Vorteilhaft wird ein Aminoglycosid zunächst in die freie Base überführt und in einem geeigneten Lösungsmittel wie Wasser oder Alkohol, insbesondere Methanol oder Ethanol oder in Lösungsmittelgemischen bestehend aus Wasser und Alkoholen mit Alkylgruppen-haltigen (HO)ₙP(O)-Säuren umgesetzt. Das Aminoglycosid und die Alkyl-haltigen (HO)ₙP(O)-Säure können dazu in entsprechenden Lösungsmitteln, beispielsweise Methanol oder Ethanol, gelöst und diese Lösungen gemischt werden. Nach der Präzipitation der phosphorhaltigen Aminoglycosid-Verbindungen können diese entweder durch Zentrifugation vom Lösungsmittel abgetrennt und im Vakuum getrocknet werden. Alternativ können die Lösungen / Suspensionen bereits vor der Trocknung direkt zur Beschichtung geeigneter glatter oder rauer Oberflächen verwendet werden.

Die Überführung des Aminoglycosids in seine freie Base erfolgt vorzugsweise mit Hilfe eines Ionenaustauschers.

In einer bevorzugten Ausführungsform wird ein Aminoglycosid, vorzugsweise Gentamicin, Streptomycin oder Kanamycin mit Dodecylphosphat in einem molaren Verhältnis des jeweiligen Aminoglycosids zu Dodecylphosphat von 1:20 bis 10:1, bevorzugt 1:7 bis 1:1, umgesetzt zu Gentamicin-Dodecylphosphat, Streptomycin-Dodecylphosphat oder Kanamycin-Dodecylphosphat.

In einer weiter vorteilhaften Ausführungsform kann ein Aminoglycosid wie Gentamicin, Streptomycin oder Kanamycin mit Octadecylphosphonsäure zu Gentamicin-Octadecylphosphonat, Streptomycin-Octadecylphosphonat oder Kanamycin-Octadecylphosphonat umgesetzt werden. Das molare Verhältnis des Aminoglycosids zu Octadecylphosphonsäure kann hierfür 1:20 bis 10:1, bevorzugt 1:5 bis 1:1 betragen.

Weiter vorteilhaft wird erfindungsgemäß Vancomycin-Dodecylphosphat hergestellt. Das molare Verhältnis von Vancomycin : Dodecylphosphat kann 1:20 bis 10:1, bevorzugt 1:8 bis 1:1, betragen.

Ferner bevorzugt ist die Herstellung von Vancomycin-Octadecylphosphonat, wobei das molare Verhältnis von Vancomycin zu Octadecylphosphonsäure zwischen 1:20 bis 10:1, bevorzugt 1:4 und 1:2, liegen kann.

Alternativ kann eine erfindungsgemäße Aminoglycosid-Verbindung gemäß Formel 1 durch Vorlegen des Aminoglycosids und Umsetzen des Aminoglycosids mit einer Alkylgruppen-haltigen (HO)ₙP(O)-Säure in Gegenwart eines Alkylamins bereitgestellt werden.

Insbesondere kann ein Gentamicin-Octadecylamin-Phosphorsäure-Salz hergestellt werden. Dafür kann zunächst Gentamicin-Base in Methanol gelöst und mit einer 25-45°C warmen Lösung aus Octadecylamin und Methanol, wobei das molare Verhältnis von Gentamicin-Base zu Octadecylamin 1:1 bis 1:4 betragen kann, gemischt. Zu der resultierenden Suspension kann Phosphorsäure in Lösung zugefügt werden. Das molare Verhältnis von Gentamicin-Base zu Octadecylamin zu Phosphorsäure kann 1:1:1 bis 1:4:4 betragen. Besonders bevorzugt ist ein molares Verhältnis von 1:2:3 bis 1:3:3.

Die biologische Aktivität der in den erfindungsgemäßen Salzen enthaltenen Aminoglycoside kann durch den Hemmhoftest mit Bacillus subtilis (ATCC 6633) bestimmt werden. Ferner kann durch den Hemmhoftest mit Bacillus subtilis (ATCC 6633) das Freisetzungsprofil der Aminoglycoside aus den erfindungsgemäßen Aminoglycosid-Salzen / -Verbindungen bestimmt werden.

### Verwendung

Die erfindungsgemäßen Aminoglycosid-Verbindungen werden vorteilhaft als injizierbares, lokales Antibiotika-Depot verwendet. Das retardierende Freisetzungsprofil ohne / mit bestimmbarem Burst release resultiert in einer besonders guten Verträglichkeit sowie in langfristig konstanten Wirkstoffspiegeln.

Ferner werden die erfindungsgemäßen Aminoglycosid-Salze vorteilhaft zur Beschichtung von Implantaten mit glatten, rauen oder strukturierten Oberflächen bereitgestellt.

Die Beschichtung geeigneter glatter, rauer oder strukturierter Oberflächen, insbesondere die Beschichtung von Implantaten erfolgt durch ein Verfahren umfassend a) Vorlegen eines Aminoglycosids, b) Umsetzen des Aminoglycosids mit einer Alkylgruppenhaltigen (HO)nP(O)-Säure, c) Erhalten eines Aminoglycosid-Salzes, d) Bereitstellung einer zu beschichtenden Oberfläche und e) Verwendung des Aminoglycosid-Salzes in fester oder gelöster Form zur physikalischen oder chemischen Vakuumbeschichtung, nasschemischen Auftrags- und Oxidationsverfahren oder Pulverauftragsverfahren, umfassend Sprüh-, Tauch- oder Tropfverfahren, wobei das Aminoglycosid-Salz auf eine zu beschichtende Oberfläche aufgebracht wird.

Die Aminoglycosid-Verbindungen gemäß der vorliegenden Erfindung eignen sich zur antibakteriellen und / oder zellwachstumsfördernden Beschichtung von Implantaten.

### Medizinische Verwendung

Die Verbindungen nach Formel 1 können vorteilhaft als Medikament verwendet werden. Durch die Anwendung der erfindungsgemäßen Aminoglyocsid-Verbindungen werden Krankheiten verschiedener Art vermieden und / oder geheilt.

### Weitere medizinische Verwendung

Besonders vorteilhaft können die erfindungsgemäßen Aminoglycosid-Verbindungen zur Behandlung von Infektionen und / oder zur Förderung des Einwachsens von Implantaten verwendet werden. Dies ist besonders vorteilhaft in Individuen welche einem Risiko für Infektionen unterliegen oder bereits unter Infektionen leiden. Des Weiteren profitieren Patienten, welche auf ein Implantat angewiesen sind, also Unfallpatienten (Knochenbrüche, Amputation etc.), Tumorpatienten, Patienten mit kardiologischen Leiden etc. von den erfindungsgemäßen Aminoglycosid-Verbindungen.

Infektionen umfassen das aktive oder passive Eindringen, Verbleiben und Vermehren von pathogenen Lebewesen oder Molekülen (Mikroorganismen), im Speziellen von Bakterien, Pilzen, Parasiten oder Viren, in einem Organismus.

Aminoglycoside hemmen den Stoffwechsel verschiedener Mikroorganismen, insbesondere verhindern sie das Wachstum, die Vermehrung und / oder das Überleben von Bakterien.

Das vorteilhafte retardierende Freisetzungsprofil der Aminoglycoside aus den erfindungsgemäßen Aminoglycosid-Verbindungen sichert therapeutisch wirksame Wirkstoffspiegel, welche langfristig konstant verfügbar sind, und erlaubt dadurch die Behandlung und / oder Prävention von Infektionen.

Besonders im Bereich der Implantologie kommt es häufig zu post-operativen Infektionen mit Erregern verschiedener Art, welche beispielsweise Mikrofilme auf der Implantatoberfläche bilden und somit den Empfänger-Organismus massiv schädigen. Derartige Infektionen können durch die vorliegenden Aminoglycosid-Verbindungen erfolgreich bekämpft und / oder verhindert werden.

Ferner weisen die erfindungsgemäßen Aminoglycosid-Verbindungen nur geringe Zytotoxizität auf und fördern das Zellwachstum, d.h. sie unterstützen folglich das Wachstum der Zellen des Empfänger-Organismus, insbesondere das Wachstum von Knochenzellen. Insbesondere steigern die Aminoglycosid-Verbindungen das Verwachsen der mit den Aminoglycosid-Verbindungen beschichteten Implantate mit dem Gewebe des Empfänger-Organismus. Der Genesungsprozess des Empfänger-Organismus wird dadurch verkürzt während die Stabilität und Akzeptanz des Implantats gesteigert wird.

Durch Implantate umfassend die erfindungsgemäßen Aminoglycosid-Verbindungen können ferner Knochen und Weichteildefekte behandelt werden.

Nachfolgend werden Ausführungsbeispiele beschrieben. Die nachstehenden Beispiele dienen der näheren Erläuterung der Erfindung, ohne diese jedoch einzuschränken.

### BEISPIELE

### Beispiel 1

### Herstellung von Gentamicin-Base

Zur Bildung der in Wasser gering löslichen Salze des Gentamicins mit den aufgeführten Alkyl-haltigen (HO)ₙP(O)-Säuren muss das Gentamicin in Form seiner Base vorliegen. Dazu wird Gentamicinsulfat mit Hilfe eines Ionenaustauschers in Gentamicin-Base überführt.

5,7 g Gentamicinsulfat wird in einem Becherglas bei Raumtemperatur in 600 ml Wasser gelöst und nach Zugabe von 50 g des Ionenaustauschers Amberlite® IRA-400 (OH) 18 Stunden bei Raumtemperatur gerührt. Danach wird der Ionenaustauscher durch Filtration entfernt und die Lösung am Rotationsverdampfer unter Vakuum auf ca. 200 ml eingeengt. Nach dem Einfrieren dieser Lösung wird eine Gefriertrocknung durchgeführt. Die daraus resultierenden weißen Flocken (3,06 g, Gentamicin-Base) werden anschließend 4 Stunden lang unter Hochvakuum (weniger als 1 mbar) bei Raumtemperatur getrocknet.

### Beispiel 2

### Umsetzung von Gentamicin mit Dodecylphosphat (molares Verhältnis 1:5)

Für die Herstellung eines Gentamicin-Dodecylphosphat-Salzes werden 100 mg Gentamicin-Base in 5 ml Methanol gelöst und mit einer Lösung aus 288 mg (1,081 mmol) Dodecylphosphat (C₁₂H₂₅OP(O)(OH)₂) in 5 ml Methanol gemischt. Das molare Verhältnis von Gentamicin-Base zu Dodecylphosphat beträgt in dieser Mischung 1:5. Zu dieser klaren Mischung werden 5 ml Wasser zugegeben und der Ansatz 30 min bei Raumtemperatur gerührt. Der sich dabei bildende Niederschlag wird nach Zugabe von 10 ml Aceton 30 min bei Raumtemperatur gerührt. Nach Zentrifugation (14.000 U/min; 5 min) wird der klare Überstand abgenommen und der feine Niederschlag im Exsikkator unter Vakuum (ca. 10 mbar) mindestens 18 h bei Raumtemperatur getrocknet. Man erhält ein Gentamicin-Dodecylphosphat-Salz mit einer Ausbeute von 67,8 % der Theorie und einem Gehalt an Gentamicin-Base von 18,5 Gew.-%. Die Konzentration der Gentamicin-Base wird mittels Hemmhoftest mit Bacillus subtilis (ATCC 6633) bestimmt.

### Beispiel 3

### Umsetzung von Gentamicin mit Dodecylphosphat (molares Verhältnis 1:4)

Analog Beispiel 2 werden 100 mg Gentamicin-Base in 5 ml Methanol gelöst und mit einer Lösung aus 229,8 mg Dodecylphosphat in 5 ml Methanol im molaren Verhältnis von 1:4 gemischt. Bei einer Ausbeute von 69,3 % der Theorie erhält man ein Gentamicin-Dodecylphosphat-Salz mit einem Gehalt an Gentamicin-Base von 19,4 Gew.-%.

### Beispiel 4

### Umsetzung von Gentamicin mit Dodecylphosphat (molares Verhältnis 1:3)

Analog Beispiel 2 werden 100 mg Gentamicin-Base in 5 ml Methanol gelöst und mit einer Lösung aus 172,4 mg Dodecylphosphat in 5 ml Methanol im molaren Verhältnis von 1:3 gemischt. Man erhält Gentamicin-Dodecylphosphat-Salz mit einem Gehalt an Gentamicin-Base von 21,4 Gew.-% (Ausbeute: 68,4 % der Theorie).

### Beispiel 5

### Umsetzung von Gentamicin mit Dodecylphosphat (molares Verhältnis 1:2)

Analog Beispiel 2 werden 100 mg Gentamicin-Base in 5 ml Methanol gelöst und mit einer Lösung aus 114,9 mg Dodecylphosphat in 5 ml Methanol im molaren Verhältnis von 1:2 gemischt. Dadurch erhält man bei einer Ausbeute von 60,4 % der Theorie ein Gentamicin-Dodecylphosphat-Salz mit einem Gehalt an Gentamicin-Base von 22,5 Gew.-%.

### Beispiel 6

### Umsetzung von Gentamicin mit Octadecylphosphonsäure (molares Verhältnis 1:5)

Zur Herstellung eines Gentamicin-Octadodecylphosphonsäure-Salzes wird eine klare Lösung aus 100 mg Gentamicin-Base in 5 ml Methanol bei einer Temperatur von 55 °C hergestellt. Nach Zugabe dieser Lösung zu einer klaren Lösung aus 360,8 mg (1,079 mmol) Octadecylphosphonsäure (C₁₈H₃₇P(O)(OH)₂) in 10 ml Methanol bei 55 °C im molaren Verhältnis von 1:5, bildet sich eine feiner Niederschlag. Zur Vervollständigung der Präzipitation wird 30 min bei Raumtemperatur gerührt und der Niederschlag anschließend durch Zentrifugation (5 min bei 14.000 U/min) abgetrennt. Danach wird der Niederschlag im Exsikkator unter Vakuum (ca. 10 mbar) mindestens 18 h bei Raumtemperatur getrocknet. Man erhält bei einer Ausbeute von 83,9 % der Theorie ein Gentamicin-Octadodecylphosphonsäure-Salz mit einem Gehalt an Gentamicin-Base von 13,3 Gew.-%.

### Beispiel 7

### Umsetzung von Gentamicin mit Octadecylphosphonsäure (molares Verhältnis 1:4)

Analog Beispiel 6 wird eine Lösung aus 100 mg Gentamicin-Base in 5 ml Methanol hergestellt. Nach Zugabe dieser Lösung zu einer Lösung aus 288,6 mg Octadecylphosphonsäure in 10 ml Methanol im molaren Verhältnis von 1:4 erhält man ein Gentamicin-Octadodecylphosphonsäure-Salz mit einem Gehalt an Gentamicin-Base von 18,2 Gew.-% und einer Ausbeute von 85,6 % der Theorie.

### Beispiel 8

### Umsetzung von Gentamicin mit Octadecylphosphonsäure (molares Verhältnis 1:3)

Analog Beispiel 6 wird eine Lösung aus 100 mg Gentamicin-Base in 5 ml Methanol hergestellt. Nach Zugabe dieser Lösung zu einer Lösung aus 216,5 mg Octadecylphosphonsäure in 10 ml Methanol im molaren Verhältnis von 1:3 erhält man ein Gentamicin-Octadodecylphosphonsäure-Salz mit einem Gehalt an Gentamicin-Base von 17,7 Gew.-% (Ausbeute 84,3 % der Theorie).

### Beispiel 9

### Umsetzung von Gentamicin mit Octadecylphosphonsäure (molares Verhältnis 1:2)

Analog Beispiel 6 wird eine Lösung aus 100 mg Gentamicin-Base in 5 ml Methanol hergestellt. Nach Zugabe dieser Lösung zu einer Lösung aus 144,3 mg Octadecylphosphonsäure in 10 ml Methanol im molaren Verhältnis von 1:2 erhält man Gentamicin-Octadodecylphosphonsäure-Salz mit einem Gehalt an Gentamicin-Base von 18,1 Gew.-%. Die Ausbeute betrug hier 70,8 % der Theorie.

### Beispiel 10

### Umsetzung von Gentamicin mit Octadecylamin und Phosphorsäure (molares Verhältnis 1:2:3)

Für die Herstellung eines Gentamicin-Octadecylamin-Phosphorsäure-Salzes werden 20 mg Gentamicin-Base in 1 ml Methanol gelöst und mit einer 35 °C warmen, klaren Lösung aus 23,3 mg (0,08625 mmol) Octadecylamin (C₁₈H₃₇NH₂) in 1 ml Methanol gemischt. Das molare Verhältnis von Gentamicin-Base zu Octadecylamin beträgt in dieser Mischung 1:2. Dabei bildet sich ein feiner Niederschlag. Zu dieser Suspension werden 14,9 mg (0,15224 mmol) Phosphorsäure als 85 %-ige Lösung zugegeben und der Ansatz 2 Stunden bei Raumtemperatur gerührt, wobei die Niederschlagsmenge zunimmt. Nach Zentrifugation des Ansatzes (14.000 U/min; 5 min) wird der klare Überstand abgenommen und der Niederschlag im Exsikkator unter Vakuum (ca. 10 mbar) mindestens 18 h bei Raumtemperatur getrocknet. Man erhält ein Gentamicin-Octadecylamin-Phosphorsäure-Salz mit einem Gehalt an Gentamicin-Base von 28,6 Gew.-%, der im Hemmhoftest mit Bacillus subtilis (ATCC 6633) bestimmt wurde (Ausbeute: 83,8 % der Theorie).

### Beispiel 11

### Umsetzung von Gentamicin mit Octadecylamin und Phosphorsäure (molares Verhältnis 1:3:3)

Analog Beispiel 10 wird eine Lösung aus 20 mg Gentamicin-Base in 1 ml Methanol vorgelegt und eine auf 35 °C erwärmte Lösung aus 34,9 mg (0,12942 mmol) Octadecylamin (C₁₈H₃₇NH₂) in 3,5 ml Methanol zugegeben. In dieser Mischung mit einem molaren Verhältnis von Gentamicin-Base zu Octadecylamin von 1:3 bildet sich sofort ein Niederschlag. Nach Zugabe von 14,9 mg (0,15224 mmol) Phosphorsäure als 85 %-ige Lösung wird ein Gentamicin-Octadecylamin-Phosphorsäure-Salz mit einer Ausbeute von 80,8 % der Theorie und einem Gehalt an Gentamicin-Base von 20,5 Gew.-% erhalten.

### Beispiel 12

### Umsetzung von Vancomycin mit Dodecylphosphat (molares Verhältnis 1:8)

Für die Herstellung eines Vancomycin-Dodecylphosphat-Salzes werden 20 mg (0,0138 mmol) Vancomycin Hydrochlorid in 2,8 ml eines Ethanol-WasserGemisches (bestehend aus 2 ml Ethanol und 0,8 ml Wasser) und 29,4 mg (0,11054 mmol) Dodecylphosphat in 0,4 ml Ethanol gelöst (molares Verhältnis von Vancomycin zu Dodecylphosphat in den Lösungen 1:8). Nach dem Mischen der beiden Lösungen und Zugabe von 5,2 ml Wasser zu dem Ansatz bildet sich ein weißer Niederschlag, der weitere 2 Stunden gerührt wird. Der Niederschlag wird durch Zentrifugation (14.000 U/min; 5 min) abgetrennt und im Exsikkator unter Vakuum (ca. 10 mbar) mindestens 18 h bei Raumtemperatur getrocknet. Dadurch erhält man ein Vancomycin-Dodecylphosphat-Salz mit einem Gehalt an Vancomycin von 0,7 Gew.-%, der im Hemmhoftest mit Bacillus subtilis (ATCC 6633) bestimmt wurde (Ausbeute: 68,1 % der Theorie).

### Beispiel 13

### Umsetzung von Vancomycin mit Dodecylphosphat (molares Verhältnis 1:6)

Analog Beispiel 12 werden 10 mg Vancomycin Hydrochlorid in 1,4 ml einer Mischung aus 1 ml Ethanol und 0,4 ml Wasser gelöst und mit einer Lösung aus 11 mg Dodecylphosphat in 0,15 ml Ethanol im molaren Verhältnis von 1:6 gemischt. Nach Zugabe von 2,6 ml Wasser und 0,05 ml Ethanol wird ein Vancomycin-Dodecylphosphat-Salz in einer Ausbeute von 56,4 % der Theorie mit einem Gehalt an Vancomycin von 1,5 Gew.-% erhalten.

### Beispiel 14

### Umsetzung von Vancomycin mit Dodecylphosphat (molares Verhältnis 1:4)

Analog Beispiel 12 werden 10 mg Vancomycin Hydrochlorid in 1,4 ml einer Mischung aus 1 ml Ethanol und 0,4 ml Wasser gelöst und mit einer Lösung aus 7,4 mg Dodecylphosphat in 0,1 ml Ethanol im molaren Verhältnis von 1:4 gemischt. Nach Zugabe von 2,6 ml Wasser sowie 0,1 ml Ethanol wird ein Vancomycin-Dodecylphosphat-Salz in einer Ausbeute von 59,4 % der Theorie und einem Gehalt an Vancomycin von 0,7 Gew.-% erhalten.

### Beispiel 15

### Umsetzung von Vancomycin mit Dodecylphosphat (molares Verhältnis 1:2)

Analog Beispiel 12 werden 10 mg Vancomycin Hydrochlorid in 1,4 ml einer Mischung aus 1 ml Ethanol und 0,4 ml Wasser gelöst und mit einer Lösung aus 3,7 mg Dodecylphosphat in 0,05 ml Ethanol im molaren Verhältnis von 1:2 gemischt. Nach Zugabe von 2,6 ml Wasser und 0,15 ml Ethanol wird ein Vancomycin-Dodecylphosphat-Salz in einer Ausbeute von 36,5 % der Theorie sowie einem Gehalt an Vancomycin von 1,2 Gew.-% erhalten.

### Beispiel 16

### Umsetzung von Vancomycin mit Dodecylphosphat (molares Verhältnis 1:1)

Analog Beispiel 12 werden 20 mg Vancomycin Hydrochlorid in 2,8 ml einer Mischung aus 2 ml Ethanol und 0,8 ml Wasser gelöst und mit einer Lösung aus 3,7 mg Dodecylphosphat in 0,05 ml Ethanol im molaren Verhältnis von 1:1 gemischt. Nach Zugabe von 5,2 ml Wasser und 0,35 ml Ethanol wird ein Vancomycin-Dodecylphosphat-Salz in einer Ausbeute von 12,3 % der Theorie erhalten.

### Beispiel 17

### Umsetzung von Vancomycin mit Octadecylphosphonsäure (molares Verhältnis 1:4)

Um ein Vancomycin-Octadodecylphosphonsäure-Salz herzustellen, wird eine Lösung aus 10 mg Vancomycin Hydrochlorid in 1,4 ml einer Ethanol-Wasser-Mischung (bestehend aus 1 ml Ethanol und 0,4 ml Wasser) vorgelegt. 9,2 mg Octadecylphosphonsäure (0,02763 mmol) werden in 0,2 ml Ethanol bei 40 °C klar gelöst und beide Lösungen gemischt, wobei sich ein Niederschlag bildet. Der Ansatz, mit einem molaren Verhältnis von Vancomycin zu Octadecylphosphonsäure von 1:4, wird weitere 2 Stunden bei Raumtemperatur gerührt und der Niederschlag durch Zentrifugation (14.000 U/min; 5 min) abgetrennt. Anschließend wird der Niederschlag im Exsikkator unter Vakuum (ca. 10 mbar) mindestens 18 h bei Raumtemperatur getrocknet. Es wird ein Vancomycin-Octadodecylphosphonsäure-Salz in einer Ausbeute von 53,3 % der Theorie sowie einem Gehalt an Vancomycin von 10,1 Gew.-% erhalten. Der Gehalt an Vancomycin wurde im Hemmhoftest mit Bacillus subtilis (ATCC 6633) bestimmt.

### Beispiel 18

### Umsetzung von Vancomycin mit Octadecylphosphonsäure (molares Verhältnis 1:2)

Analog Beispiel 17 werden 20 mg Vancomycin Hydrochlorid in 2,8 ml einer Mischung aus 2 ml Ethanol und 0,8 ml Wasser gelöst und mit einer bei 40 °C klaren Lösung aus 9,2 mg Octadecylphosphonsäure (0,02763 mmol) in 0,2 ml Ethanol im molaren Verhältnis von 1:2 gemischt. Dabei wird ein Vancomycin-Octadecylphosphonsäure-Salz in einer Ausbeute von 56,7 % der Theorie mit einem Gehalt an Vancomycin von 0,7 Gew.-% erhalten.

### Beispiel 19

### Umsetzung von Streptomycin mit Dodecylphosphat (molares Verhältnis 1:7)

Zur Herstellung eines Streptomycin-Dodecylphosphat-Salzes werden 20 mg (0,02745 mmol) Streptomycinsulfat in 1 ml Wasser sowie 53,5 mg (0,20077 mmol) Dodecylphosphat in 1 ml Ethanol gelöst. Nach dem Mischen der beiden Lösungen mit einem molaren Verhältnis von Streptomycin zu Dodecylphosphat von 1:7 und Zugabe von 2 ml Wasser zu dem Ansatz bildet sich ein weißer Niederschlag, der weitere 2 Stunden bei Raumtemperatur gerührt wird. Der Niederschlag wird durch Zentrifugation (14.000 U/min; 5 min) abgetrennt und im Exsikkator unter Vakuum (ca. 10 mbar) mindestens 18 h bei Raumtemperatur getrocknet. Das so erhaltene Streptomycin-Dodecylphosphat-Salz weist einem Gehalt an Streptomycin von 1,9 Gew.-%, der im Hemmhoftest mit Bacillus subtilis (ATCC 6633) bestimmt wurde (Ausbeute: 76,0 % der Theorie), auf.

### Beispiel 20

### Umsetzung von Streptomycin mit Dodecylphosphat (molares Verhältnis 1:4)

Analog Beispiel 19 werden 20 mg Streptomycinsulfat in 1 ml Wasser gelöst und mit einer Lösung aus 30,6 mg Dodecylphosphat in 0,5713 ml Ethanol im molaren Verhältnis von Streptomycin zu Dodecylphosphat von 1:4 gemischt. Nach Zugabe von 0,4287 ml Ethanol sowie 2 ml Wasser wird ein Streptomycin-Dodecylphosphat-Salz in einer Ausbeute von 61,4 % der Theorie und einem Gehalt an Streptomycin von 4,7 Gew.-% erhalten.

### Beispiel 21

### Umsetzung von Streptomycin mit Dodecylphosphat (molares Verhältnis 1:2)

Analog Beispiel 19 werden 20 mg Streptomycinsulfat in 1 ml Wasser gelöst und mit einer Lösung aus 15,3 mg Dodecylphosphat in 0,2858 ml Ethanol im molaren Verhältnis von Streptomycin zu Dodecylphosphat von 1:2 gemischt. Nach Zugabe von 0,7142 ml Ethanol sowie 2 ml Wasser wird ein Streptomycin-Dodecylphosphat-Salz in einer Ausbeute von 34,2 % der Theorie und einem Gehalt an Streptomycin von 5,4 Gew.-% erhalten.

### Beispiel 22

### Umsetzung von Streptomycin mit Dodecylphosphat (molares Verhältnis 1:1)

Analog Beispiel 19 werden 20 mg Streptomycinsulfat in 1 ml Wasser gelöst und mit einer Lösung aus 7,6 mg Dodecylphosphat in 0,1427 ml Ethanol im molaren Verhältnis von Streptomycin zu Dodecylphosphat von 1:1 gemischt. Nach Zugabe von 0,8573 ml Ethanol sowie 2 ml Wasser wird ein Streptomycin-Dodecylphosphat-Salz in einer Ausbeute von 13,6 % der Theorie und einem Gehalt an Streptomycin von 2,1 Gew.-% erhalten.

### Beispiel 23

### Herstellung von Kanamycin-Base

Entsprechend dem Beispiel 1 werden in einem Becherglas 0,57 g Kanamycinsulfat in 60 ml Wasser bei Raumtemperatur gelöst. Nach Zugabe von 5 g des Ionenaustauschers Amberlite® IRA-400 (OH) wird 18 h bei Raumtemperatur gerührt. Anschließend wird der Ionenaustauscher durch Filtration entfernt und die Lösung am Rotationsverdampfer unter Vakuum auf ca. 20 ml eingeengt. Nach dem Einfrieren dieser Lösung wird eine Gefriertrocknung durchgeführt. Das dabei erhaltene Produkt (0,45 g) wird 4 h lang unter Hochvakuum (weniger als 1 mbar) bei Raumtemperatur getrocknet. Die biologische Aktivität der so gewonnenen Kanamycin-Base wird im Hemmhoftest mit Bacillus subtilis (ATCC 6633) bestimmt.

### Beispiel 24

### Umsetzung von Kanamycin mit Dodecylphosphat (molares Verhältnis 1:4)

Für die Herstellung eines Kanamycin-Dodecylphosphat-Salzes werden 10 mg (0,02064 mmol) Kanamycin-Base in 1 ml Wasser sowie 22 mg (0,08261 mmol) Dodecylphosphat in 0,5 ml Ethanol gelöst und die beiden Lösungen mit einem molaren Verhältnis von Kanamycin-Base zu Dodecylphosphat von 1:4 gemischt. Der sich nach Zugabe von 0,5 ml Ethanol und 2 ml Wasser bildende weiße Niederschlag wird weitere 2 h bei Raumtemperatur gerührt. Nach dem Abtrennen des Niederschlags durch Zentrifugation (14.000 U/min; 5 min) wird dieser im Exsikkator unter Vakuum (ca. 10 mbar) mindestens 18 h bei Raumtemperatur getrocknet. Das daraus resultierende Kanamycin-Dodecylphosphat-Salz wird in einer Ausbeute: 27,8 % der Theorie gewonnen und besitzt einen Gehalt an Kanamycin-Base von 23,8 Gew.-% (bestimmt im Hemmhoftest mit Bacillus subtilis (ATCC 6633)).

### Beispiel 25

### Umsetzung von Kanamycin mit Dodecylphosphat (molares Verhältnis 1:2)

Analog Beispiel 24 werden 10 mg Kanamycin-Base in 1 ml Wasser gelöst und mit einer Lösung aus 11 mg Dodecylphosphat (0,0413 mmol) in 0,25 ml Ethanol im molaren Verhältnis von Kanamycin zu Dodecylphosphat von 1:2 gemischt. Nach Zugabe von 0,75 ml Ethanol und 1 ml Wasser wird ein Kanamycin-Dodecylphosphat-Salz in einer Ausbeute von 41,6 % der Theorie und einem Gehalt an Kanamycin-Base von 45,9 Gew.-% erhalten.

### Beispiel 26

### Umsetzung von Kanamycin mit Dodecylphosphat (molares Verhältnis 1:1)

Analog Beispiel 24 werden 10 mg Kanamycin-Base in 1 ml Wasser gelöst und mit einer Lösung aus 5,5 mg Dodecylphosphat (0,02065 mmol) in 0,125 ml Ethanol im molaren Verhältnis von Kanamycin zu Dodecylphosphat von 1:1 gemischt. Nach Zugabe von 0,875 ml Ethanol und 1 ml Wasser wird ein Kanamycin-Dodecylphosphat-Salz in einer Ausbeute von 30,6 % der Theorie und einem Gehalt an Kanamycin-Base von 44,7 Gew.-% erhalten.

### Beispiel 27

### Umsetzung von Kanamycin mit Octadecylphosphonsäure (molares Verhältnis 1:4)

Ein Vancomycin-Octadodecylphosphonsäure-Salz wird generiert, indem eine Lösung aus 10 mg Kanamycin-Base (0,02064 mmol) in 1 ml einer Mischung aus Ethanol und Wasser (60 % Ethanol und 40 % Wasser) hergestellt wird. Nachdem 27,6 mg Octadecylphosphonsäure (0,08252 mmol) in 1 ml Ethanol bei 50 °C klar gelöst sind, werden beide Lösungen mit einem molaren Verhältnis von Vancomycin zu Octadecylphosphonsäure von 1:4 gemischt. Der sich bildende Niederschlag wird weitere 2 h bei Raumtemperatur gerührt, durch Zentrifugation (14.000 U/min; 5 min) abgetrennt und im Exsikkator unter Vakuum (ca. 10 mbar) mindestens 18 h bei Raumtemperatur getrocknet. Dadurch kann ein Kanamycin-Octadodecylphosphonsäure-Salz mit einer Ausbeute von 91,0 % der Theorie und einem Gehalt an Vancomycin von 25,0 Gew.-% gewonnen werden. Der Gehalt an Kanamycin-Base wurde im Hemmhoftest mit Bacillus subtilis (ATCC 6633) bestimmt.

### Beispiel 28

### Umsetzung von Kanamycin mit Octadecylphosphonsäure (molares Verhältnis 1:2)

Analog Beispiel 27 werden 10 mg Kanamycin-Base in 1 ml einer Mischung aus 0,6 ml Ethanol und 0,4 ml Wasser gelöst und mit einer bei 50 °C klaren Lösung aus 13,9 mg Octadecylphosphonsäure (0,04144 mmol) in 1 ml Ethanol im molaren Verhältnis von 1:2 gemischt. Dabei wird ein Kanamycin-Octadecylphosphonsäure-Salz in einer Ausbeute von 83,6 % der Theorie mit einem Gehalt an Kanamycin-Base von 30,1 Gew.-% erhalten.

### Beispiel 29

### Umsetzung von Kanamycin mit Octadecylphosphonsäure (molares Verhältnis 1:1)

Analog Beispiel 27 werden 10 mg Kanamycin-Base in 1 ml einer Mischung aus 0,6 ml Ethanol und 0,4 ml Wasser gelöst und mit einer bei 50 °C klaren Lösung aus 6,9 mg Octadecylphosphonsäure (0,02069 mmol) in 1 ml Ethanol im molaren Verhältnis von 1:1 gemischt. Dabei wird ein Kanamycin-Octadecylphosphonsäure-Salz in einer Ausbeute von 60,2 % der Theorie mit einem Gehalt an Kanamycin-Base von 34,7 Gew.-% erhalten.

### Beispiel 30

### Freisetzung von Aminoglycosiden aus Beschichtungen

Zur Untersuchung der Freisetzung von Aminoglykosiden aus Beschichtungen werden runde Probekörper aus Reintitan (Grade 4) mit einem Durchmesser von 11 mm verwendet. Für die Beschichtungen werden folgende Aminoglycosid-Verbindungen verwendet: Gentamicin-Palmitat, Gentamicin-Dodecylphosphat und Gentamicin-Octadecylphosphonsäure.

### Herstellung der Aminoglycosid-Verbindungen

Umsetzung von Gentamicin mit Palmitat im molaren Verhältnis 1:4. Umsetzung von Gentamicin mit Dodecylphosphat bzw. Octadecylphosphonat im molaren Verhältnis 1:3.

### Beschichtung der Probekörper mit Gentamicin-Palmitat, Gentamicin-Dodecylphosphat und Gentamicin-Octadecylphosphonsäure

Lösung von Gentamicin-Palmitat, Gentamicin-Dodecylphosphat und Gentamicin-Octadecylphosphonsäure in Ethanol (Konzentration 15-25 mg/ml). Auf die Oberfläche wurden unter sterilen Bedingungen 100 µg Gentamicin-Base pro cm² aufgetragen.
Trocknen der Beschichtung (4 h bei Raumtemperatur).

### Freisetzung der Aminoglycoside

Inkubation der beschichteten Probekörper in 1 ml isotonischer Kochsalzlösung über eine Zeitdauer von 21 d bei 37 °C. Zu festgelegten Zeitpunkten (1, 2, 3, 4, 7, 14 und 21 Tagen) werden die Überstände abgenommen und die Probekörper in neuer isotonischer Kochsalzlösung weiter inkubiert.

Der Gehalt an Gentamicin wurde im Hemmhoftest mit Bacillus subtilis (ATCC 6633) bestimmt.

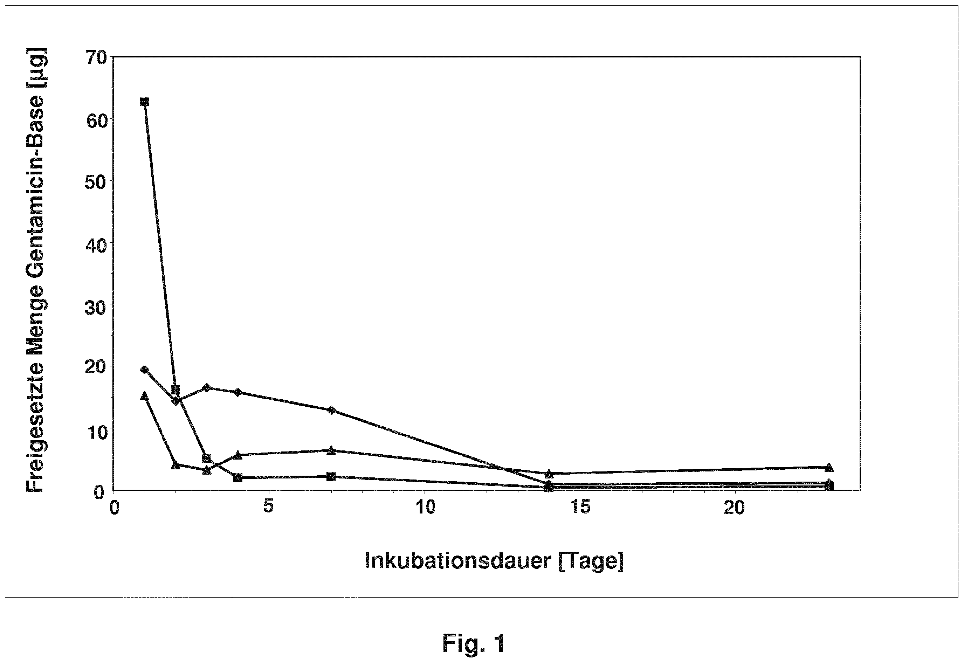

Die gemittelte Menge der freigesetzten Gentamicin-Base aus diesen Gentamicin-Salzen ist in Fig. 1 dargestellt. Gentamicin-Palmitat (■), Gentamicin-Dodecylphosphat (◆) und Gentamicin-Octadecylphosphonsäure (▲). y-Achse: freigesetzte Konzentration an Gentamicin-Base in µg; x-Achse: Inkubationsdauer in Tagen.

In Fig. 1 sind bezüglich der Freisetzung der Gentamicin-Base deutliche Unterschiede der drei verwendeten Gentamicin-Salze zu erkennen. Gentamicin-Palmitat (■) führt zu einer initial hohen Freisetzung der Gentamicin-Base (Burst release), die bis zum vierten Freisetzungstag stark abnimmt.
Gentamicin-Dodecylphosphat (◆) zeigt zu Beginn eine niedrigere Freisetzungsrate von Gentamicin-Base als das Gentamicin-Palmitat. Die Freisetzungrate bleibt bis Tag 7 konstant und fällt dann nur schwach ab. Gentamicin-Octadecylphosphonsäure (▲) zeigt im Vergleich zu den anderen beiden Salzen die niedrigste initiale Freisetzung von Gentamicin-Base. Die Wirkstofffreisetzung bleibt von Tag 2 bis Tag 14 konstant.

Fig. 1 zeigt also deutlich die retardierende Wirkstofffreisetzung durch die erfindungsgemäßen Verbindungen Gentamicin-Dodecylphosphat und Gentamicin-Octadecylphosphonat von beschichteten Titanoberflächen im Vergleich zu Gentamicin-Palmitat, wobei die Wirkstofffreisetzung langfristig über mehrere Tage bis Wochen konstant bleibt.

### Beispiel 31

### Untersuchungen der Cytokompatibilität aminoglykosidhaltiger Beschichtungen

Für die Untersuchungen der Cytokompatibilität der aminoglykosidhaltigen Beschichtungen werden runde, sandgestrahlte Probekörper aus Reintitan mit einem Durchmesser von 11 mm mit Gentamicin-Palmitat, Gentamicin-Dodecylphosphat bzw. Gentamicin-Octadecylphosphonsäure mit einem Gehalt von 100 µg Gentamicn-Base pro cm², wie in Beispiel 30 beschrieben, beschichtet.

Die Probekörper werden nach dem Trocknen der Beschichtung in Mikrotiterplatten überführt und mit einer definierten Anzahl von MC 3T3-E1-Zellen (Maus-Präosteoblasten-Zelllinie, 25.000 Zellen/ml) besiedelt.

Nach einer Inkubationszeit von 4 Tagen erfolgt die Vitalfärbung mit einer Mischung aus Fluoresceindiacetat (FDA) und GelRed®.

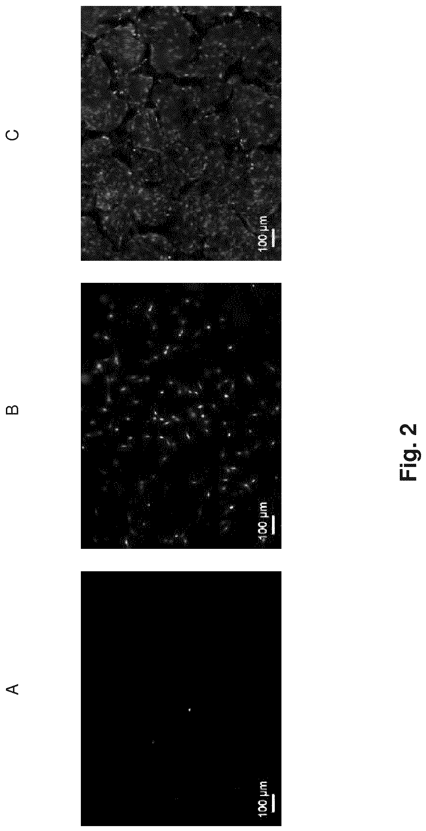

Fig. 2 zeigt die Vitalitätsbestimmung der Zellen mittels Auflicht-Fluoreszenzmikroskopie: Gentamicin-Palmitat (A), Gentamicin-Dodecylphosphat (B), Gentamicin-Octadecylphosphat (C).

Beschichtung mit Getnaimcin-Palmitat (A) führt zu abgerundeten Zellen und, im Vergleich zu den beiden anderen Beschichtungen, zu den niedrigsten Zellzahlen.
Gentamicin-Dodecylphosphat (B) ist durch signifikant geringere Zytotoxizität charakterisiert. Die untersuchten Zellen sind adhärent und vital.
Gleiches ist nach Inkubation der Zellen mit Gentamicin-Octadecylphosphonat (C) zu beobachten.

Die Erfindung ist nicht auf die erläuterten Ausführungsbeispiele beschränkt. Alle beschriebenen, beanspruchten und/oder gezeichneten Merkmale können im Rahmen der Erfindung beliebig miteinander kombiniert werden.

## Patentansprüche

1. Verbindung der Formel 1 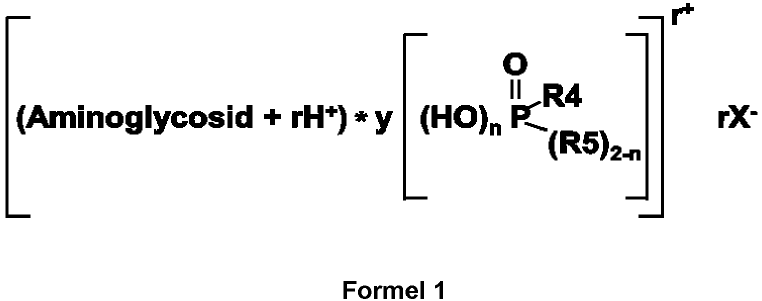
- wobei das Aminoglycosid ausgewählt ist aus der Gruppe der Aminoglycosid- oder Aminoglycosidgruppen-haltigen Antibiotika,
- wobei y ausgewählt ist aus 0,1 bis 20,
- wobei n ausgewählt ist aus 1 oder 2,
- wobei r ausgewählt ist aus 0, 1, 2 bis 5,
- wobei X ausgewählt ist aus der Gruppe bestehend aus Halogenen, HCOO, CH₃COO, CH₃SO₃, Phosphatresten, oder Sulfatresten,
- wobei R4 ausgewählt ist aus der Gruppe bestehend aus -C₁-C₃₀-Alkyl, - C₂-C₃₀-Alkenyl, -C₂-C₃₀-Alkinyl, -O-(C₄-C₃₀-Alkandiyl)phosphat, -(C₄-C₃₀-Alkandiyl)phosphonat, -O-R6, -O-P(O)(OH)(O-R6), -O-P(O)(-O-R6)(-O-R7), -O-P(O)(-R6)(-O-R7), -O⁻[NR8,R9,R10,H]⁺, -O-P(O)(-R6)(-O⁻ [NR8,R9,R10,H]⁺), -O-P(O)(-O-R6)(-O⁻[NR8,R9,R10,H]⁺),
- wobei R5 ausgewählt ist aus der Gruppe bestehend aus -C₁-C₃₀-Alkyl,-C₂-C₃₀-Alkenyl, -C₂-C₃₀-Alkinyl, -O-(C₄-C₃₀-Alkandiyl)phosphat, -(C₄-C₃₀-Alkandiyl)phosphonat, -O-R6, -O-P(O)(OH)(O-R6), -O-P(O)(-O-R6)(-O-R7), -O-P(O)(-R6)(-O-R7), -O⁻[NR8,R9,R10,H]⁺, -O-P(O)(-R6)(-O⁻ [NR8,R9,R10,H]⁺), -O-P(O)(-O-R6)(-O⁻[NR8,R9,R10,H]⁺), oder wobei n 1 und R5 H ist,
- wobei R6, R7 und R10 unabhängig ausgewählt sind aus der Gruppe-C₁-C₃₀-Alkyl, -C₂-C₃₀-Alkenyl oder -C₂-C₃₀-Alkinyl,
- wobei R8, R9 unabhängig ausgewählt sind aus der Gruppe -C₁-C₃₀-Alkyl, -C₂-C₃₀-Alkenyl oder -C₂-C₃₀-Alkinyl, oder wobei R8 und R9 unabhängig voneinander H sind.

2. Verbindung gemäß Anspruch 1,
**dadurch gekennzeichnet,**
**dass** y ausgewählt ist aus 0.33, 1, 2, 2.5, 3, 4 oder 5.

3. Verbindung gemäß einem der vorhergehenden Ansprüchen,
**dadurch gekennzeichnet,**
**dass** X ausgewählt ist aus Chlor, Fluor, Iod oder Brom, vorzugsweise aus Chlor oder Brom.

4. Verbindung gemäß einem der vorhergehenden Ansprüche,
**dadurch gekennzeichnet,**
**dass** die Gruppen -C₁-C₃₀-Alkyl, -C₂-C₃₀-Alkenyl, -C₂-C₃₀-Alkinyl zwei oder mehrere ungesättigte C-Atome enthalten, verzweigt oder unverzweigt sind, oder mit Halogenatomen oder organischen funktionellen Gruppen substituiert sind, wobei das Halogenatom vorzugsweise Fluor oder Chlor ist.

5. Verbindung gemäß einem der vorhergehenden Ansprüche,
**dadurch gekennzeichnet,**
**dass** das Aminoglycosid- oder Aminoglycosidgruppen-haltige Antibiotikum ausgewählt ist aus der Gruppe bestehend aus Amikacin, Apramycin, Geneticin, Kanamycin, Netilmicin, Neomycin, Paromomycin, Spectinomycin, Streptomycin, Tobramycin, Gentamicin, oder Vancomycin.

6. Verbindung gemäß einem der vorhergehenden Ansprüche,
**dadurch gekennzeichnet,**
**dass** die Verbindung ausgewählt ist aus der Gruppe bestehend aus Gentam icin-Dodecylphosphat, Gentam icin-Octadecylphosphonat, Vancomycin-Dodecylphosphat, Vancomycin-Octadecylphosphonat, Streptomycin-Dodecylphosphat, Kanamycin-Dodecylphosphat, Kanamycin-Octadecylphosphonat, vorzugsweise Gentamicin-Dodecylphosphat oder Gentam icin-Octadecylphosphonat ist.

7. Verbindung gemäß einem der vorhergehenden Ansprüche,
**dadurch gekennzeichnet,**
**dass** das Aminoglycosid retardierend freigesetzt wird.

8. Verbindung gemäß einem der vorhergehenden Ansprüche,
**dadurch gekennzeichnet,**
**dass** sie das Zellwachstum fördert.

9. Verbindung gemäß einem der vorhergehenden Ansprüche,
**dadurch gekennzeichnet,**
**dass** sie in gelöster oder fester Form vorliegt.

10. Zusammensetzung
**dadurch gekennzeichnet,**
**dass** die Zusammensetzung mindestens 2, 3, 4, 5, 6, 7, 8, 9, 10, 11, oder 12 verschiedene Verbindungen gemäß den vorhergehenden Ansprüchen umfasst.

11. Zusammensetzung gemäß Anspruch 10,
**dadurch gekennzeichnet,**
**dass** die Zusammensetzung mindestens 2, 3, 4, 5, 6, 7, 8, 9, 10, 11, oder 12 verschiedene Aminoglycoside umfasst ausgewählt aus der Gruppe bestehend aus Amikacin, Apramycin, Geneticin, Kanamycin, Netilmicin, Neomycin, Paromomycin, Spectinomycin, Streptomycin, Tobramycin, Gentamicin, oder Vancomycin.

12. Verfahren zur Herstellung einer Verbindung gemäß einem der Ansprüche 1 bis 9,
**dadurch gekennzeichnet,**
**dass** es die folgenden Schritte umfasst:
a) Vorlegen eines Aminoglycosids,
b) Umsetzen des Aminoglycosids mit einer Alkylgruppen-haltigen (HO)ₙP(O)-Säure,
c) Erhalten eines Aminoglycosid-Salzes.

13. Verfahren zur Herstellung einer Verbindung gemäß Anspruch 12,
**dadurch gekennzeichnet,**
**dass** in Schritt b) das Umsetzen in Gegenwart einer Base und Alkali- oder Erdalkalisalzen einer Alkylgruppen-haltigen (HO)ₙP(O)-Säure erfolgt.

14. Verfahren zur Herstellung einer Verbindung gemäß Anspruch 12,
**dadurch gekennzeichnet,**
**dass** in Schritt a) ein Aminoglycosid oder ein Aminoglycosid-Salz vorgelegt wird, in Schritt b) das Umsetzen mit Alkylgruppen-haltigen (HO)ₙP(O)-Säuren in Gegenwart eines Alkylamins erfolgt.

15. Verbindung gemäß einem der Ansprüche 1 bis 9 oder einer Zusammensetzung gemäß einem der Ansprüche 10 bis 11
a) zur Anwendung als injizierbares, lokales Antibiotika-Depot,
b) zur antibakteriellen Beschichtung von Implantaten und / oder
c) zur zellwachstumsfördernden Beschichtung von Implantaten.

16. Verbindung gemäß einem der Ansprüche 1 bis 9 oder Zusammensetzung gemäß einem der Ansprüche 10 bis 11 zur Verwendung als Medikament.

17. Verbindung gemäß einem der Ansprüche 1 bis 9 oder Zusammensetzung gemäß einem der Ansprüche 10 bis 11
a) zur Behandlung von Infektionen und / oder
b) zur Förderung des Einwachsverhaltens von Implantaten.

18. Implantat,
**dadurch gekennzeichnet,**
**dass** es eine Verbindung gemäß einem der Ansprüche 1 bis 9 oder eine Zusammensetzung gemäß einem der Ansprüche 10 bis 11 umfasst.

19. Implantat gemäß Anspruch 18 zur Behandlung von Knochen- oder Weichteildefekten.

20. Antibakterielle Beschichtung,
**dadurch gekennzeichnet,**
**dass** sie eine Verbindung gemäß einem der Ansprüche 1 bis 9 oder eine Zusammensetzung gemäß einem der Ansprüche 10 bis 11 umfasst.

## Claims

1. Compound of formula 1 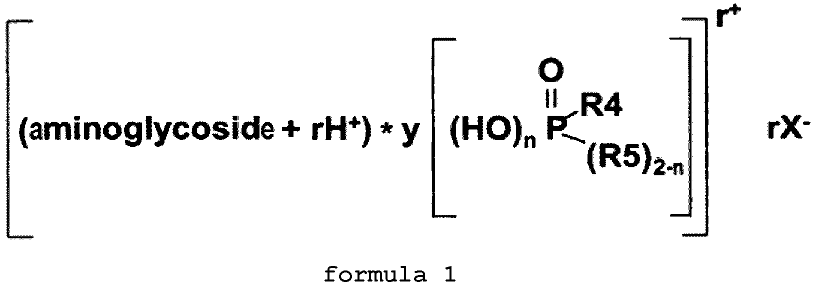
- wherein the aminoglycoside is selected from the group of the aminoglycoside antibiotics or aminoglycoside group-containing antibiotics,
- wherein y is selected from 0.1 to 20,
- wherein n is selected from 1 or 2,
- wherein r is selected from 0, 1, 2 to 5,
- wherein X is selected from the group consisting of halogens, HCOO, CH₃COO, CH₃SO₃, phosphate residue or sulfate residue,
- wherein R4 is selected from the group consisting of -C₁-C₃₀-alkyl, -C₂-C₃₀-alkenyl, -C₂-C₃₀-alkynyl , -O-(C₄-C₃₀-alkanediyl) phosphate, -(C₄-C₃₀-alkanediyl) phosphonate, -O-R6, -O-P(O)(OH)(O-R6), -O-P(O)(-O-R6)(-O-R7), -O-P(O)(-R6)(-O-R7), -O⁻[NR8,R9,R10 ,H]⁺, -O-P(O)(-R6)(-O⁻[NR8,R9,R10,H]⁺), -O-P(O)(-O-R6)(-O⁻[NR8,R9,R10,H]⁺),
- wherein R5 is selected from the group consisting of -C₁-C₃₀-alkyl, -C₂-C₃₀-alkenyl, -C₂-C₃₀-alkynyl, -O-(C₄-C₃₀-alkanediyl) phosphate, -(C₄-C₃₀-alkanediyl) phosphonate, -O-R6, -O-P(O)(OH)(O-R6), -O-P(O)(-O-R6)(-O-R7), -O-P(O)(-R6)(-O-R7), -O⁻[NR8,R9,R10 ,H]⁺, -O-P(O)(-R6)(-O⁻[NR8,R9,R10,H]⁺), -O-P(O)(-O-R6)(-O⁻[NR8,R9,R10,H]⁺), or wherein n is 1 and R5 is H,
- wherein R6, R7 and R10 are independently selected from the group of -C₁-C₃₀-alkyl, -C₂-C₃₀-alkenyl or -C₂-C₃₀-alkynyl,
- wherein R8, R9 are independently selected from the group of -C₁-C₃₀-alkyl, -C₂-C₃₀-alkenyl or -C₂-C₃₀-alkynyl, or wherein R8 and R9 are each independently H.

2. Compound according to Claim 1,
**characterized**
**in that** y is selected from 0.33, 1, 2, 2.5, 3, 4 or 5.

3. Compound according to either of the preceding claims,
**characterized**
**in that** X is selected from chlorine, fluorine, iodine or bromine, preferably from chlorine or bromine.

4. Compound according to any of the preceding claims,
**characterized**
**in that** the groups -C₁-C₃₀-alkyl, -C₂-C₃₀-alkenyl, -C₂-C₃₀-alkynyl contain two or more unsaturated carbon atoms, are branched or unbranched, or are substituted by halogen atoms or organic functional groups, wherein the halogen atom is preferably fluorine or chlorine.

5. Compound according to any of the preceding claims,
**characterized**
**in that** the aminoglycoside antibiotic or aminoglycoside group-containing antibiotic is selected from the group consisting of amikacin, apramycin, geneticin, kanamycin, netilmicin, neomycin, paromomycin, spectinomycin, streptomycin, tobramycin, gentamicin, or vancomycin.

6. Compound according to any of the preceding claims,
**characterized**
**in that** the compound is selected from the group consisting of gentamicin dodecylphosphate, gentamicin octadecylphosphonate, vancomycin dodecylphosphate, vancomycin octadecylphosphonate, streptomycin dodecylphosphate, kanamycin dodecylphosphate, kanamycin octadecylphosphonate, preferably is gentamicin dodecylphosphate or gentamicin octadecylphosphonate.

7. Compound according to any of the preceding claims,
**characterized**
**in that** the aminoglycoside is released in a sustained manner.

8. Compound according to any of the preceding claims,
**characterized**
**in that** it promotes cell growth.

9. Compound according to any of the preceding claims,
**characterized**
**in that** it is present in dissolved or solid form.

10. Composition
**characterized**
**in that** the composition comprises at least 2, 3, 4, 5, 6, 7, 8, 9, 10, 11 or 12 different compounds according to the preceding claims.

11. Composition according to Claim 10,
**characterized**
**in that** the composition comprises at least 2, 3, 4, 5, 6, 7, 8, 9, 10, 11 or 12 different aminoglycosides selected from the group consisting of amikacin, apramycin, geneticin, kanamycin, netilmicin, neomycin, paromomycin, spectinomycin, streptomycin, tobramycin, gentamicin, or vancomycin.

12. Process for preparing a compound according to any of Claims 1 to 9,
**characterized**
**in that** it comprises the following steps:
a) providing an aminoglycoside,
b) reacting the aminoglycoside with an alkyl group-containing (HO)ₙP(O) acid,
c) obtaining an aminoglycoside salt.

13. Process for preparing a compound according to Claim 12,
**characterized**
**in that** in step b) the reaction is effected in the presence of a base and alkali metal salts or alkaline earth metal salts of an alkyl group-containing (HO)ₙP(O) acid.

14. Process for preparing a compound according to Claim 12,
**characterized**
**in that** in step a) an aminoglycoside or an aminoglycoside salt is provided, in step b) the reaction with alkyl group-containing (HO)ₙP(O) acids is effected in the presence of an alkylamine.

15. Compound according to any of Claims 1 to 9 or a composition according to either of Claims 10 and 11
a) for application as an injectable, local antibiotic depot,
b) for the antibacterial coating of implants and/or
c) for the cell growth-promoting coating of implants.

16. Compound according to any of Claims 1 to 9 or composition according to either of Claims 10 and 11 for use as a medication.

17. Compound according to any of Claims 1 to 9 or composition according to either of Claims 10 and 11
a) for treating infections and/or
b) for promoting the incorporation behaviour of implants.

18. Implant
**characterized**
**in that** it comprises a compound according to any of Claims 1 to 9 or a composition according to either of Claims 10 and 11.

19. Implant according to Claim 18 for the treatment of bone or soft tissue defects.

20. Antibacterial coating,
**characterized**
**in that** it comprises a compound according to any of Claims 1 to 9 or a composition according to either of Claims 10 and 11.

## Revendications

1. Composé de formule 1 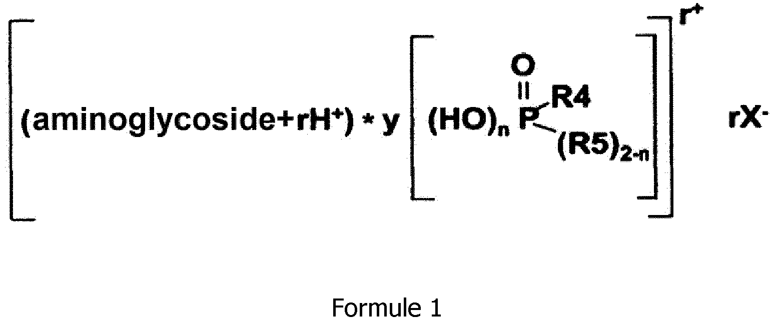
- où l'aminoglycoside est choisi dans le groupe des antibiotiques aminoglycosides ou contenant des groupes aminoglycosides,
- où y est choisi parmi 0, 1 à 20,
- où n est choisi parmi 1 ou 2,
- où r est choisi parmi 0, 1, 2 à 5,
- où X est choisi dans le groupe consistant en les halogènes, HCOO, CH₃COO, CH₃SO₃, les groupements phosphate ou les groupements sulfate,
- où R4 est choisi dans le groupe consistant en -C₁-C₃₀-alkyle, -C₂-C₃₀-alcényle, -C₂-C₃₀-alcynyle, -O-(C₄-C₃₀-alcanediyl)phosphate, -(C₄-C₃₀-alcanediyl)phosphonate, -O-R6, -O-P(O)(OH)(O-R6), -O-P(O)(-O-R6)(-O-R7), -O-P(O)(-R6)(-O-R7), -O⁻[NR8,R9,R10,H]⁺, -O-P(O)(-R6)(-O⁻[NR8,R9,R10,H]⁺), -O-P(O)(-O-R6)(-O⁻ [NR8,R9,R10,H]⁺),
- où R5 est choisi dans le groupe consistant en -C₁-C₃₀-alkyle, -C₂-C₃₀-alcényle, -C₂-C₃₀-alcynyle, -O-(C₄-C₃₀-alcanediyl)phosphate, -(C₄-C₃₀-alcanediyl)phosphonate, -O-R6, -O-P(O)(OH)(O-R6), -O-P(O)(-O-R6)(-O-R7), -O-P(O)(-R6)(-O-R7), -O⁻[NR8,R9,R10,H]⁺, -O-P(O)(-R6)(-O⁻[NR8,R9,R10,H]⁺), -O-P(O)(-O-R6)(-O⁻ [NR8,R9,R10,H]⁺), ou bien où n est 1 et R5 est H,
- où R6, R7 et R10 sont choisis indépendamment dans le groupe -C₁-C₃₀-alkyle, -C₂-C₃₀-alcényle ou -C₂-C₃₀-alcynyle,
- où R8, R9 sont choisis indépendamment dans le groupe -C₁-C₃₀-alkyle, -C₂-C₃₀-alcényle ou -C₂-C₃₀-alcynyle, ou bien où R8 et R9 sont indépendamment l'un de l'autre H.

2. Composé selon la revendication 1, **caractérisé en ce que** y est choisi parmi 0,33, 1, 2, 2,5, 3, 4 ou 5.

3. Composé selon l'une des revendications précédentes, **caractérisé en ce que** X est choisi parmi le chlore, le fluor, l'iode ou le brome, de préférence parmi le chlore ou le brome.

4. Composé selon l'une des revendications précédentes, **caractérisé en ce que** les groupes -C₁-C₃₀-alkyle, -C₂-C₃₀-alcényle, -C₂-C₃₀-alcynyle contiennent deux ou plusieurs atomes C insaturés, sont ramifiés ou non ramifiés, ou sont substitués avec des atomes d'halogène ou des groupes fonctionnels organiques, où l'atome d'halogène est de préférence le fluor ou le chlore.

5. Composé selon l'une des revendications précédentes, **caractérisé en ce que** l'antibiotique aminoglycoside ou contenant des groupes aminoglycosides est choisi dans le groupe consistant en l'amikacine, l'apramycine, la généticine, la kanamycine, la nétilmicine, la néomycine, la paromomycine, la spectinomycine, la streptomycine, la tobramycine, la gentamicine ou la vancomycine.

6. Composé selon l'une des revendications précédentes, **caractérisé en ce que** le composé est choisi dans le groupe consistant en le dodécylphosphate de gentamicine, l'octadécylphosphonate de gentamicine, le dodécylphosphate de vancomycine, l'octadécylphosphonate de vancomycine, le dodécylphosphate de streptomycine, le dodécylphosphate de kanamycine, l'octadécylphosphonate de kanamycine, de préférence est le dodécylphosphate de gentamicine ou l'octadécylphosphonate de gentamicine.

7. Composé selon l'une des revendications précédentes, **caractérisé en ce que** l'aminoglycoside est libéré de manière retardatrice.

8. Composé selon l'une des revendications précédentes, **caractérisé en ce qu'**il favorise la croissance cellulaire.

9. Composé selon l'une des revendications précédentes, **caractérisé en ce qu'**il est présent sous forme dissoute ou solide.

10. Composition, **caractérisée en ce que** la composition comprend au moins 2, 3, 4, 5, 6, 7, 8, 9, 10, 11 ou 12 composés selon les revendications précédentes différents.

11. Composition selon la revendication 10, **caractérisée en ce que** la composition comprend au moins 2, 3, 4, 5, 6, 7, 8, 9, 10, 11 ou 12 aminoglycosides différents choisis dans le groupe consistant en l'amikacine, l'apramycine, la généticine, la kanamycine, la nétilmicine, la néomycine, la paromomycine, la spectinomycine, la streptomycine, la tobramycine, la gentamicine ou la vancomycine.

12. Procédé pour la préparation d'un composé selon l'une des revendications 1 à 9, **caractérisé en ce qu'**il comprend les étapes suivantes :
a) disposition préalable d'un aminoglycoside,
b) réaction de l'aminoglycoside avec un acide (HO)ₙP(O)-contenant des groupes alkyle,
c) obtention d'un sel d'aminoglycoside.

13. Procédé pour la préparation d'un composé selon la revendication 12, **caractérisé en ce que**, dans l'étape b), la réaction a lieu en présence d'une base et de sels alcalins ou alcalino-terreux d'un acide (HO)ₙP(O)- contenant des groupes alkyle.

14. Procédé pour la préparation d'un composé selon la revendication 12, **caractérisé en ce que**, dans l'étape a), un aminoglycoside ou un sel d'aminoglycoside est disposé au préalable, dans l'étape b), la réaction avec des acides (HO)ₙP(O)- contenant des groupes alkyle a lieu en présence d'une alkylamine.

15. Composé selon l'une des revendications 1 à 9 ou composition selon l'une des revendications 10 à 11
a) destiné à être utilisé comme forme retard d'antibiotique locale injectable,
b) pour le revêtement antibactérien d'implants et/ou
c) pour le revêtement favorisant la croissance cellulaire d'implants.

16. Composé selon l'une des revendications 1 à 9 ou composition selon l'une des revendications 10 à 11 destiné à être utilisé comme médicament.

17. Composé selon l'une des revendications 1 à 9 ou composition selon l'une des revendications 10 à 11
a) pour le traitement d'infections et/ou
b) pour favoriser le comportement de croissance d'implants.

18. Implant, **caractérisé en ce qu'**il comprend un composé selon l'une des revendications 1 à 9 ou une composition selon l'une des revendications 10 à 11.

19. Implant selon la revendication 18 pour le traitement de défauts osseux ou de tissus mous.

20. Revêtement antibactérien **caractérisé en ce qu'**il comprend un composé selon l'une des revendications 1 à 9 ou une composition selon l'une des revendications 10 à 11.
